# EUROPÄISCHE PATENTSCHRIFT

(11) **EP 2 938 365 B1**
(45) Veröffentlichungstag und Bekanntmachung des Hinweises auf die Patenterteilung: **21.12.2016**
(21) Anmeldenummer: 14771938.9
(22) Anmeldetag: 24.09.2014
(51) Int. Cl.: A61L 2/20, B65D 88/12

(54) **BEGEHBARE STATIONÄRE ETHYLENOXID-STERILISATIONSANLAGE**
STATIONARY ETHYLENE OXIDE STERILISATION INSTALLATION
INSTALLATION DE STÉRILISATION STATIONNAIRE À L'OXYDE D'ÉTHYLÈNE

(30) Priorität: 25.09.2013 EP 13186047
(43) Veröffentlichungstag der Anmeldung: 04.11.2015
(73) Patentinhaber: B. Braun Melsungen AG, 34212 Melsungen (DE)
(72) Erfinder: GEIGER, Ralph, 34587 Felsberg (DE)
(74) Vertreter: Winter, Brandl, Fürniss, Hübner, Röss, Kaiser, Polte - Partnerschaft mbB
(86) Internationale Anmeldenummer: PCT/EP2014/070416
(87) Internationale Veröffentlichungsnummer: WO 2015/044233

(56) Entgegenhaltungen:
- WO-A1-2013/124274
- CN-A- 103 386 141
- US-A- 3 436 173
- US-A- 3 490 863
- US-A- 5 891 390
- R. Salzbrunn, R.M. Macmerth: "Ethylenoxid-Gassterisiation" In: Kramer, Assadian: "Praxis der Sterilisation, Desinfektion, Antiseptik und Konservierung", 31. Dezember 2008 (2008-12-31), Georg Thieme Verlag KG, XP002715746, ISBN: 9783131411211 Seiten 88-90, Abbildung 5.22

## Beschreibung

Die vorliegende Erfindung betrifft eine begehbare stationäre Ethylenoxid-Sterilisationsanlage umfassend eine Vakuumpumpe, einen Ethylenoxidverdampfer, einen Ethylenoxidtank oder Ethylenoxidflasche, eine Steuerung, eine Befeuchtungsanlage (Dampfgenerator) und eine Sterilisationskammer, wobei sich die Sterilisationskammer, die Vakuumpumpe, der Ethylenoxidverdampfer und der Ethylenoxidtank in einem Metallcontainer oder mehreren Metallcontainern befinden. Vakuumpumpe, Ethylenoxidverdampfer und Ethylenoxidtank befinden sich in einem Metallcontainer, wobei sich die Sterilisationskammer in demselben oder einem weiteren Metallcontainer befinden kann.

Die Gassterilisation mittels Ethylenoxid ist ein gängiges und lange etabliertes Verfahren zur Sterilisation von diversen Erzeugnissen, dessen Wirkungsweise in gängigen Standardwerken zur Sterilisation allgemein abgehandelt wird (siehe z.B. Kramer und Assadian (2008): "Praxis der Sterilisation, Desinfektion, Antiseptik und Konservierung", Kapitel 5.9, Seiten 88 bis 90). Folglich gibt es bereits viele kompakte Systeme zur Gassterilisation mittels Ethylenoxid, die für die Anwendung in einzelnen Laboratorien und Krankenhaus-Abteilungen konzipiert wurden.

So wird in der US 3 436 173 eine tragbare und eigenständige Vorrichtung zur Ethylenoxid-Gassterilisation für eine schnelle Sterilisation ohne das Risiko einer Explosion beschrieben. Die tragbare Vorrichtung umfasst in einem kompakten Gehäuse eine Sterilisationskammer mit einer speziellen explosionssicheren Tür, einen Ethylenoxid-Zerstäuber, eine elektrisch betriebene Vakuumpumpe, Steuerungseinheiten, Heizelemente, und eine von außen zugängliche und über einen Schraubverschluss dicht verschließbare Vorrichtung zur Aufnahme einer Einzeldosis-Kartusche (30 Gramm Kartusche).

Auch die Druckschrift "Ethylenoxid-Gassterilisation" von R. Salzbrunn und R. M. Macmerth legt die Grundlagen der Ethylenoxidsterilisation dar und offenbart, ein vollautomatischer Ethylenoxidsterilisator gleiche einer industriellen Anlage auf kleinem Raum.

In dem US-amerikanischen Patent (US 5 891 390) der Joslyn Sterilizer Corporation wird ein kompaktes System zur Ethylenoxid-Gassterilisation bestehend aus zwei Kompartimenten beschrieben, das eine sichere Dosierung von Ethylenoxid aus großen Gebinden für einen einzelnen Sterilisationszyklus ermöglichen und zugleich die Verwendung von kostenintensiven Einzeldosis-Kartuschen vermeiden soll. Das erste Kompartiment enthält in seinem Gehäuse die Ethylenoxid-Gebinde sowie eine Dosierungseinheit und das zweite Kompartiment enthält eine drucksichere Sterilisationskammer mit einem Volumen von 8,8 Kubikfuß (entspricht 0,25 m³), eine Vakuumpumpe und eine Befeuchtungsanlage. Zudem ist eine Vorrichtung zur Belüftung beider Kompartimente vorgesehen. Daneben ist eine Vorrichtung vorhanden, die im Fall eines Gasaustrittes die Flutung des ersten Kompartimentes mit Wasser ermöglicht und so die Brand- bzw. Explosionsgefahr reduziert.

Die US 3 490 863 beschreibt ebenfalls eine Vorrichtung zur Gassterilisation mittels Ethylenoxid bestehend aus zwei Kompartimenten, bei der die Menge an eingeleitetem Sterilisationsgas gemessen und reguliert werden soll, so dass je nach Beladungsvolumen der Sterilisationskammer nur die notwendige Menge an Gas zugeleitet wird. Das erste Kompartiment in Form einer von oben über einen Klappdeckel zugänglichen Kiste stellt die Sterilisationskammer mit einem Volumen von 3 Kubikfuß (entspricht 0,085 m³) dar, während das zweite Kompartiment das Versorgungskompartiment mit einem Aufnahmebehälter für eine Einzeldosis-Kartusche mit angeschlossenem Hitze-Verdampfer, einer Vakuumpumpe sowie Gasleitungen, Sensoren und Kontrollventile darstellt. Der Fokus der US 3 490 863 liegt auf einem Kontrollmechanismus, der bei Erreichen eines gewissen Gasdruckes in der Sterilisationskammer ein Sicherheitsventil öffnet und so den Einlass weiteren Gases in die Sterilisationskammer stoppt und so die Gefahr eines Überdrucks in der Sterilisationskammer reduziert. Gleichzeitig wird die Gaskartusche über eine Bypass-Leitung unter Umgehung der Sterilisationskammer entleert, um eine Gefährdung durch Restgas in der Kartusche zu vermeiden.

Im industriellen Maßstab verwendete Sterilisationsanlagen, d.h. Anlagen mit großen Beladungsvolumina, die für die Sterilisation von Paletten geeignet sind, stellen eine ganz andere Herausforderung dar. Bestehende industriell genutzte Ethylenoxid(EO)-Sterilisationsanlagen (Palettensterilisationsanlagen) werden vor Ort beim Betreiber endmontiert. Hierbei entsteht ein erheblicher Aufwand während der Installation und Inbetriebnahme. Die Versorgungseinheiten sind in verschiedenen Räumen untergebracht und werden über Rohrleitungen miteinander verbunden.

Eine derartige begehbare Sterilisationsanlage ist beispielsweise aus der WO 2013 / 124274 A1 bekannt. Hierbei ist für eine homogenere Verteilung des Sterilisationsgases in der Sterilisationskammer ein Mischer ohne bewegliche Teile vorgesehen.

Zusätzlich muss für die einzelnen Aufstellungsräume der Explosionsschutz gebäudeseitig mit beachtet werden. Es ergibt sich daraus zusätzlich ein erhöhter Aufwand für das EO-Monitoring, sowie für die Gebäudelüftung (explosionssichere Auslegung sowie ein stetiger Luftaustausch im Normalbetrieb in den Räumen, sowie erhöhte Notlüftung bei Leckage).

Der Erfindung liegt somit die Aufgabe zugrunde, eine begehbare, stationäre Ethylenoxid-Sterilisationsanlage, welche zur Palettensterilisation geeignet ist, in vormontierter versandfertiger Weise bereitzustellen.

Es wurde gefunden, dass diese Aufgabe gelöst wird, indem zumindest die explosionsgefährdeten Komponenten einer begehbaren, stationären Ethylenoxid-Sterilisationsanlage in einem Container (vorzugsweise einem Metallcontainer) oder mehreren Containern bzw. vorzugsweise zwei Containern weiter bevorzugt mehreren Metallcontainern bzw. besonders bevorzugt zwei Metallcontainern untergebracht werden. Zudem ist bevorzugt, wenn diese über eine Lüftungsanlage (auch als Abluftanlage, Belüftungsanlage oder Ventilation bezeichnet) versorgt werden, welche normalerweise kein Bestandteil einer Sterilisationsanlage ist.

Die vorliegende Erfindung betrifft folglich ebenfalls eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind, und wobei die begehbare stationäre Ethylenoxid-Sterilisationsanlage zusätzlich eine Lüftungsanlage zur Belüftung des/der Container sowie der enthaltenen Sterilisationskammer umfasst.

Somit betrifft die vorliegende Erfindung eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage (z.B. in Form eines Dampfgenerators, insbesondere elektrisch oder mit fossilen Brennstoffen beheizt) und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind und dieser Metallcontainer vorzugsweise über eine Lüftungsanlage (beispielsweise einen Ventilator) verfügt, welche fest mit der Kammer verbunden sein kann. Die Auslegung der Lüftungsanlage ist von dem Luftvolumen der Containerräume abhängig. Vorzugsweise ist ein zehnfacher Luftwechsel pro Stunde im Regelbetrieb zu verwenden. Diese Luftwechselrate kann auf das Doppelte im Falle einer Störung angehoben werden um z.B. austretende Gasmengen rasch zu verdünnen und zu entfernen. Die Überwachung der Lüftungsanlage sowie die Überwachung der Messwerte aus den Vorrichtungen (Sensoren) zur Überwachung des Ethylenoxid-Gehaltes (auch als "EO-Monitoring-Anlage" bzw. "Ethylenoxid-Monitoring-Anlage" bezeichnet) wird vorzugsweise durch die Steuerung (bzw. Steuervorrichtung) der Ethylenoxid-Sterilisationsanlage übernommen. Um eine effiziente Lüftung der Sterilisationskammer zu garantieren und gleichzeitig den Bereich und das Personal außerhalb des Containers vor Ethylenoxid zu schützen, werden zusätzlich die Zugänge (bzw. Türen oder Tore) des Containers (vor allem die Zugänge zur Sterilisationskammer) durch die Steuerung der Ethylenoxid-Sterilisationsanlage überwacht, d.h. der Zustand der Zugänge (offen oder geschlossen) wird von der Steuerung erfasst und überwacht. Somit ist die räumliche Überwachung des Sterilisationsbereiches bevorzugt in die Steuerung der Ethylenoxid-Sterilisationsanlage integriert. Dampfgeneratoren mit hohen Heizleistungen können in unterschiedlichen Stufen zugeschaltet werden (z.B. 60 kW in 4 Stufen a 15 kW). Die je nach konkreter Anwendung benötigte Heizleistung des Dampfgenerators ist abhängig von der Größe der Sterilisationskammer und dem zu sterilisierenden Produkt und kann somit stark variieren.

Die Befeuchtung der zu sterilisierenden Produkte ist ein wichtiger Bestandteil des Sterilisationsprozesses. Die Befeuchtung wird teilweise schon vor dem eigentlichen Sterilisationsprozesses durchgeführt und kann in einem separaten Raum erfolgen. Da diese Variante jedoch zusätzlichen Platz benötigt, wird diese in der vorliegenden Erfindung nicht bevorzugt. In der vorliegenden Erfindung wird ein Dampfgenerator eingesetzt, welcher über eine ausreichende Kapazität für eine direkte Befeuchtung im Sterilisationsprozess verfügt. Die Erzeugung des Dampfes für den eigentlichen Sterilisationsprozess erfolgt im Allgemeinen über das Verdampfen von Wasser, bevorzugt destilliertes oder vollentsalztes Wasser. Die Verdampfung erfolgt über einen Temperaturanstieg.

Eine Sterilisationsanlage zur Palettensterilisation, wie hierin verwendet bedeutet, dass die Sterilisationsanlage technisch dazu ausgelegt ist, nicht nur einzelne Produkte zu sterilisieren, sondern dass ganze Paletten, die mit zu sterilisierenden Produkten beladen sind, in die Sterilisationsanlage geladen werden und dort sterilisiert werden können.

Die vorliegende Erfindung betrifft daher ebenfalls eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind, wobei es sich bei der begehbaren stationären Ethylenoxid-Sterilisationsanlage um eine Palettensterilisationsanlage handelt.

Werden in der vorliegenden Beschreibung in Klammern Angaben zu den dreidimensionalen Maßen eines Gegenstandes (z.B. eines Metallcontainers) gemacht, beziehen sich diese (wenn nicht anders erwähnt) auf die Außenmaße des Gegenstandes und sind in der Form "X m x Y m x Z m" verfasst, wobei diese Bezeichnung der Reihenfolge Länge x Breite x Höhe (L x B x H) folgt. Das beispielhafte Objekt A (10 m x 3 m x 1,5 m) hat also eine Länge von 10 Metern eine Breite von 3 Metern und ist 1,5 Meter hoch, während das beispielhafte Objekt B (5 m x 3 m x 3 m) zwar genauso breit aber nur halb so lang und dafür doppelt so hoch wie Objekt A ist.

Als "Paletten", wie hierin verwendet, werden flache, in der Regel rechteckige Untersätze bezeichnet, die für die Lagerung und für den Transport größerer Mengen (stapelbarer) Waren (z.B. zu sterilisierende Produkte) verwendet werden. "Flach" bedeutet in diesem Zusammenhang, dass bezüglich der Maße des Untersatzes (definiert durch Länge, Breite und Höhe) die Höhe weniger als ein Viertel der Länge des Untersatzes beträgt. Unter den Begriff "Palette", wie hierin verwendet, fallen die gängigen Europool-Paletten (1,2 m x 0,8 m x 0,144 m, auch als Euro-Paletten bezeichnet), die halben Europool-Paletten (0,8 m x 0,6 m x 0,144 m, auch als Displaypaletten bezeichnet), die so genannten standardisierten Industrie-Paletten (1,2 m x 1,0 m x 0,144 m), nicht-standardisierte Industriepaletten (2,0 m x 1,2 m x 0,144 m oder 2,0 m x 1,25 m x 0,144 m) sowie die im US-amerikanischen und chinesischen Raum gängigen Paletten (ca. 1,2 m x 1,0 m x 0,140 m) als auch die im sonstigen asiatischen Raum gängigen Paletten (1,1 m x 1,1 m x 0,13 m bzw. 1,14 m x 1,14 m x 0,13 m). Es versteht sich von selbst, dass es bei den Maßen der verwendeten Paletten auch herstellungsbedingt geringfügige Abweichungen geben kann. Paletten werden in der Regel nicht per Hand sondern unter Zuhilfenahme von Flurfördergeräten wie z.B. Hubwagen, Langläufer oder Gabelstapler transportiert.

Aus den oben gemachten Ausführungen ist ersichtlich, dass die erfindungsgemäße Ethylenoxid-Sterilisationsanlage zur Beladung von Personal in der Regel mit einem Flurfördergerät begangen wird. Es handelt sich also bei der erfindungsgemäßen Ethylenoxid-Sterilisationsanlage um eine **begehbare** Ethylenoxid-Sterilisationsanlage. Dies wird auch aus den weiter unten aufgeführten Maßen und Volumina der Anlage ersichtlich.

Somit betrifft die vorliegende Erfindung ebenfalls eine begehbare Ethylenoxid-Sterilisationsanlage umfassend eine Vakuumpumpe, einen Ethylenoxidverdampfer, einen Ethylenoxidtank, eine Steuerung, eine Befeuchtungsanlage (z.B. in Form eines Dampfgenerators, insbesondere elektrisch oder mit fossilen Brennstoffen beheizt) und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind.

Somit betrifft die vorliegende Erfindung ebenfalls eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage (z.B. in Form eines Dampfgenerators, insbesondere elektrisch oder mit fossilen Brennstoffen beheizt) und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind.

Die vorliegende Erfindung ist ebenfalls gerichtet auf eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage (z.B. in Form eines Dampfgenerators, insbesondere elektrisch oder mit fossilen Brennstoffen beheizt) und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind, und wobei die begehbare stationäre Ethylenoxid-Sterilisationsanlage des Weiteren Rohrleitungen zwischen Sterilisationskammer und Vakuumpumpe, zwischen Sterilisationskammer und Ethylenoxidverdampfer, zwischen Ethylenoxidverdampfer und Ethylenoxidtank sowie zwischen Sterilisationskammer und Befeuchtungsanlage umfasst.

Wie bereits aus den oben gemachten Ausführungen ersichtlich, können bei den erfindungsgemäßen Ethylenoxid-Sterilisationsanlagen verschiedene Kompartimente innerhalb des Metallcontainers bzw. der Metallcontainer unterschieden werden. Ein Kompartiment stellt die Sterilisationskammer dar, während ein zweiter oder weiteres Kompartiment, welches auch als "Technikraum" bezeichnet werden kann, den Raum begrifflich abgrenzt, indem die weiteren technischen Komponenten der Anlage (wie z.B. Vakuumpumpe, Ethylenoxidverdampfer, Ethylenoxidtank, Steuerung, Befeuchtungsanlage, Lüftungsanlage) untergebracht sind. Wenngleich natürlich dieser Technikraum z.B. für den Austausch leerer Ethylenoxidtanks oder Wartungsarbeiten idealerweise durch Wartungspersonal begehbar sein sollte, so bezieht sich das Attribut "begehbar" in Verbindung mit der erfindungsgemäßen Ethylenoxid-Sterilisationsanlage vor allem auf die Sterilisationskammer, die vom Personal mit zu sterilisierenden Produkten (in der Regel zu Paletten zusammengefasst) beladen werden muss.

Somit betrifft die vorliegende Erfindung ebenfalls eine Ethylenoxid-Sterilisationsanlage umfassend eine Vakuumpumpe, einen Ethylenoxidverdampfer, einen Ethylenoxidtank, eine Steuerung, eine Befeuchtungsanlage (z.B. in Form eines Dampfgenerators, insbesondere elektrisch oder mit fossilen Brennstoffen beheizt) und eine begehbare Sterilisationskammer, dadurch gekennzeichnet, dass die begehbare Sterilisationskammer, die Vakuumpumpe, der Ethylenoxidverdampfer und der Ethylenoxidtank in mindestens einem Metallcontainer untergebracht sind.

Unter dem Begriff "mindestens einem Metallcontainer" soll verstanden werden, dass sich die vorgenannten Komponenten in einem Metallcontainer befinden, sich aber auch auf mehrere Metallcontainer und vorzugsweise auf zwei Metallcontainer verteilen können. Besonders bevorzugt ist eine Ein-Container-Variante. Werden jedoch zwei Metallcontainer verwendet, werden diese bevorzugt über einen Versorgungskanal miteinander verbunden und sind daher leicht, d.h. ohne großen Aufwand, zu montieren. Die mitgelieferte Lüftungsanlage würde dann beide Metallcontainer versorgen, bzw. entlüften und kann außerhalb der beiden Container angebracht sein, ist jedoch bevorzugt in einem der beiden Metallcontainer untergebracht. Um den Aufbau zu erleichtern, wird bei der Zwei-Container-Variante eine Justierung über eine bevorzugt abnehmbare Justierhilfe ausgerichtet.

Bei dem im Rahmen der Zwei-Container-Variante beschriebenen Versorgungskanal handelt es sich um ein Verbindungselement, welches (nach Möglichkeit) alle Leitungen beinhaltet, die eine funktionale Verbindung zwischen technischen Komponenten in einem ersten Metallcontainer der erfindungsgemäßen Ethylenoxid-Sterilisationsanlage mit technischen Komponenten in einem zweiten Metallcontainer der erfindungsgemäßen Ethylenoxid-Sterilisationsanlage herstellen. In einer bevorzugten Ausführungsform der Zwei-Container-Variante, in der in einem ersten Container die Vakuumpumpe (optional mit angeschlossener Abgasreinigungsanlage), der Ethylenoxidverdampfer, der Ethylenoxidtank sowie die Befeuchtungsanlage (z.B. in Form eines Dampfgenerators) enthalten ist und der zweite Container die Sterilisationskammer beinhaltet, sind dies die Leitung von der Vakuumpumpe zur Sterilisationskammer, die Leitung von der Befeuchtungsanlage zur Sterilisationskammer sowie die Leitung vom Ethylenoxid-Verdampfer zur Sterilisationskammer. Sind in dem ersten Container zudem ein Warmwassersystem für die Heizung der Sterilisationskammer und/oder eine Lüftungsanlage für beide Container untergebracht, so repräsentieren auch die Leitungen des Warmwassersystems und/oder der Lüftungsanlage derartige funktionale Verbindungen. Die Gesamtheit der funktionalen Verbindungen in dem Versorgungskanal wird bevorzugt durch einen Mantel, d.h. den Mantel des Versorgungskanals, umgeben, welcher einen runden, vieleckigen oder rechteckigen Querschnitt haben kann. In einer bevorzugten Ausführungsform der Erfindung ist dieser Mantel des Versorgungskanals explosionssicher ausgestaltet. Das Zusammenfassen der Leitungen zwischen zwei Containern in einem Versorgungskanal erleichtert nicht nur die Montage erheblich, sondern erhöht auch die Sicherheit.

Dementsprechend ist es bevorzugt, wenn die innere Querschnittfläche des Versorgungskanalmantels nicht mehr beträgt als das Zweifache der gesamten äußeren Querschnittsfläche aller Leitungen, die sich in dem Versorgungskanal befinden, bevorzugt nicht mehr als das 1,8-fache, weiter bevorzugt nicht mehr als das 1,5-fache und am meisten bevorzugt nicht mehr als das 1,2-fache der gesamten äußeren Querschnittsfläche aller Leitungen in dem Versorgungskanal.

In einer bevorzugten Ausführungsform der Zwei-Container-Variante befindet sich der Versorgungskanal mit den enthaltenden Leitungen bereits vormontiert in einem der beiden Container. Die entsprechenden Anschlüsse in dem zweiten Container sind ebenfalls vormontiert. Nach der Aufstellung am Zielort muss der Versorgungskanal lediglich aus besagtem Container herausgeschoben werden und mit dem zweiten Container verbunden werden. Hierbei ist es möglich, dass die Leitungen über dichte aber einfach zu bedienende Schraubverbindungen verbunden werden. Es ist natürlich auch denkbar, dass bestimmte Komponenten (wie z.B. der Mantel des Versorgungskanals) am Zielort vernietet werden. Dem Fachmann sind zahlreiche Möglichkeiten bekannt, um einfache aber sichere Verbindungen am Zielort zu ermöglichen.

Zusätzlich kann sich zur Erhöhung der Anlagensicherheit in bestimmten Ausführungsformen der vorliegenden Erfindung ein Ethylenoxid-Sensor in dem Versorgungskanal befinden, über den der Ethylenoxid-Gehalt in dem Versorgungskanal überwacht werden kann.

Gemäß einer weiteren Ausführungsform einer Zwei-Container oder auch Mehr-Container-Variante samt Versorgungskanal ist es bevorzugt, wenn der Mantel des Versorgungskanal bzw. der von ihm umschlossenen Innenraum des Versorgungskanals an die Lüftungsanlage der erfindungsgemäßen Ethylenoxid-Sterilisationsanlage angeschlossen ist, d.h. ein Teil von ihr ist. Somit würden über den Versorgungskanal nicht nur die funktionellen Verbindungen zwischen technischen Komponenten in verschiedenen Containern (z.B. Dampfgenerator und Sterilisationskammer) hergestellt, sondern zugleich der angebundene Container belüftet. Dies spart nicht nur Raum, sondern erhöht zudem die Sicherheit, da zugleich natürlich auch der Versorgungskanal belüftet wird.

Somit verteilen sich die explosionsgefährdeten Komponenten der stationären Ethylenoxid-Sterilisationsanlage nicht über verschiedene Räume, sondern befinden sich zusammen in einem oder mehreren Metallcontainern, bevorzugt jedoch in einem Metallcontainer. Bei den explosionsgefährdeten Komponenten der stationären Ethylenoxid-Sterilisationsanlage handelt es sich um die Sterilisationskammer, die Vakuumpumpe, den Ethylenoxidverdampfer und den Ethylenoxidtank. Je nach Ausführungsform der vorliegenden Erfindung, kann die Ethylenoxid-Sterilisationsanlage des Weiteren eine Abgasreinigungsanlage zur Entsorgung des toxischen Ethylenoxid umfassen, die dann ebenfalls zu den explosionsgefährdeten Komponenten der Ethylenoxid-Sterilisationsanlage gehört. Es versteht sich von selbst, dass die notwendigen Leitungen, welche die explosionsgefährdeten Komponenten der Ethylenoxid-Sterilisationsanlage miteinander verbinden, auch explosionsgefährdet sind.

Somit können die explosionsgefährdeten Komponenten der Ethylenoxid-Sterilisationsanlage, je nach Ausführungsform, die Sterilisationskammer, die Vakuumpumpe, den Ethylenoxidverdampfer, den Ethylenoxidtank, die Abgasreinigungsanlage sowie die diese Komponenten verbindenden gasführenden Leitungen beinhalten.

Die vorliegende Erfindung betrifft folglich ebenfalls eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind, und wobei sich die explosionsgefährdeten Bauteile innerhalb eines Metallcontainers befinden.

Bei dem Metallcontainer oder den Metallcontainern der vorliegenden Erfindung handelt es sich vorzugsweise um genormte Metallcontainer, auch als Seecontainer oder Frachtcontainer bezeichnet.

Gemäß einer weiteren Ausführungsform ist die vorliegende Erfindung ist folglich ebenfalls gerichtet auf eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind, wobei es sich bei dem mindestens einen Metallcontainer um einen Seecontainer (bzw. Frachtcontainer) handelt.

Eine Norm, die die Maße von Frachtcontainern festlegt, ist z.B. die mittlerweile zurückgezogene ISO-Norm 668 aus dem Jahr 1975. Container, die dieser Norm folgen, werden auch als ISO-Container bezeichnet. In Deutschland existiert zudem die DIN 15190 zur Festlegung der Maße von Frachtcontainern.

Entsprechend sind weltweit gängige und erfindungsgemäß bevorzugte Container-Längen 6,058 m, 7,150 m, 7,315 m, 7,430 m, 7,450 m, 7,820 m, 8,100 m, 9,125 m, 12,192 m, 12,500 m, 13,106 m, 13,600 m, 13,716 m, 14,630 m, 14,935 m und 16,154 m. Besonders bevorzugt werden jedoch die Container-Längen 6,058 m, 12,192 m, 13,716m und 16,154 m. Die Seite des Containers (bzw. Metallcontainers), die durch die Länge definiert wird, wird auch als Längsseite bezeichnet. Gemäß einer weiteren Ausführungsform der vorliegenden Erfindungen ist bevorzugt, wenn der erfindungsgemäße Container (bevorzugt ein Metallcontainer) eine Länge von 11,340 m aufweist.

Entsprechend sind weltweit gängige und erfindungsgemäß bevorzugte Container-Breiten 2,438 m, 2,500 m, 2,550 m und 2,591 m. Die Seite des Containers (bzw. Metallcontainers), die durch die Breite definiert wird, wird auch als Querseite bezeichnet. Gemäß einer weiteren Ausführungsform der vorliegenden Erfindungen ist bevorzugt, wenn der erfindungsgemäße Container (bevorzugt ein Metallcontainer) eine Breite von 2,658 m aufweist.

Entsprechend sind weltweit gängige und erfindungsgemäß bevorzugte Container-Höhen 2,438 m, 2,591 m, 2,743 m und 2,895 m. Gemäß einer weiteren Ausführungsform der vorliegenden Erfindungen ist bevorzugt, wenn der erfindungsgemäße Container (bevorzugt ein Metallcontainer) eine Höhe von 2,71 m bzw. eine Höhe von 3,00 m aufweist.

Folglich ist erfindungsgemäß bevorzugt, wenn es sich bei dem oder den Metallcontainern um Metallcontainer mit einer Mindestlänge von 6 m, einer Mindestbreite von 2,4 m und einer Mindesthöhe von 2,4 m handelt.

Besonders bevorzugt ist im Rahmen der vorliegenden Erfindung, wenn es sich bei dem oder den Metallcontainern um genormte Metallcontainer handelt, ausgewählt aus der Gruppe bestehen aus "20-ft-Container (klein)" (6,058 m x 2,438 m x 2,438 m), "20-ft-Container" (6,058 m x 2,438 m x 2,591 m), "20-ft-Container HC" (6,058 m x 2,438 m x 2,896 m), "30-ft-Container (klein)" (9,125 m x 2,438 m x 2,438 m), "30-ft-Container" (9,125 m x 2,438 m x 2,591 m), "40-ft-Container (klein)" (12,192 m x 2,438 m x 2,438 m), "40-ft-Container" (12,192 m x 2,438 m x 2,591 m), "40-ft-Container HC" (12,192 m x 2,438 m x 2,896 m), "45-ft-Container HC" (13,716 m x 2,438 m x 2,896 m), "45-ft-Container PW" (13,716 m x 2,49 m x 2,591 m) und "53-ft-Container HC" (16,154 m x 2,591 m x 2,896 m). Gemäß einer weiteren Ausführungsform der vorliegenden Erfindungen ist bevorzugt, wenn der erfindungsgemäße Container (bevorzugt ein Metallcontainer) eine Länge von 11,340 m, eine Breite von 2,658 m und eine Höhe von 2,71 m aufweist. Gemäß einer weiteren Ausführungsform der vorliegenden Erfindungen ist bevorzugt, wenn der erfindungsgemäße Container (bevorzugt ein Metallcontainer) eine Länge von 12,19 m, eine Breite von 2,43 m und eine Höhe von 3,00 m aufweist.

Bei der Benennung der Container steht "HC" für eine gängige höhere Container-Variante (die so genannte "*high cube*"-Variante), während "PW" für eine gängige breitere und an die Breite von Paletten angepasste Container-Variante ("*pallet wide*") steht.

Es handelt sich, wie oben bereits definiert, bei den Maßangaben zur Länge, Breite und Höhe der Container um Außenmaße (wenn nicht anders erwähnt).

Es sei an dieser Stelle klargestellt, dass auch bei den genormten Maßen eine Maßtoleranz von +/- 10 mm gewährt wird.

Das Leergewicht der erfindungsgemäß geeigneten Container sowie deren Innenvolumina hängen natürlich nicht nur von den oben angegeben Außenmaßen, sondern auch von dem Material und der Wandstärke des Containermantels ab. Als groben Richtwert haben gängige "20-ft-Container" ein Leergewicht zwischen 2,0 t und 3,0 t und ein Innenvolumen von ungefähr 33 m³, "20-ft-Container HC" ein Leergewicht zwischen 2,2 t und 3,4 t und ein Innenvolumen von ungefähr 37 m³, "40-ft-Container" ein Leergewicht zwischen 3,8 t und 5 t und ein Innenvolumen von ungefähr 67,5 m³, "40-ft-Container HC" ein Leergewicht zwischen zwischen 4 t und 8 t, insbesondere zwischen 6 t und 7 t und ein Innenvolumen von ungefähr 76 m³, "45-ft-Container HC"ein Leergewicht zwischen 4,4 t und 8 t und ein Innenvolumen von ungefähr 86 m³, "45-ft-Container PW" ein Leergewicht zwischen 4,4 t und 8 t und ein Innenvolumen von ungefähr 79 m³ und "53-ft-Container HC" ein Leergewicht zwischen 5 t und 9 t und ein Innenvolumen von ungefähr 109 m³.

Der Begriff "Volumen des Containers" oder "Container-Volumen", wie hierin verwendet, bezieht sich(wenn nicht anders erwähnt) auf das Innenvolumen und kann somit gleichbedeutend mit den Begriffen "Innenvolumen des Containers" oder "Container-Innenvolumen" verwendet werden. Es soll an dieser Stelle zudem klargestellt werden, dass das Innenvolumen des Containers lediglich das Volumen, das durch den Container-Mantel eingeschlossen wird, berücksichtigt also nicht das Volumen, das innerhalb des Containers z.B. durch Bauteile der Sterilisationsanlage (z.B. die Vakuumpumpe) eingenommen wird. Hierzu wird als Beispiel eine erfindungsgemäße Sterilisationsanlage in Gestalt eines "40-ft-Containers" (12,192 m x 2,438 m x 2,591 m) betrachtet. Dieser beispielhafte "40-ft-Container" hat die Innenmaße 12,032 m x 2,352 m x 2,385 m und somit gemäß obiger Definition ein Innenvolumen von knapp 67,5 m³, unabhängig davon, wie viele Bauteile der Sterilisationsanlage in dem Container montiert sind.

Gemäß einer weiteren Ausführungsform der vorliegenden Erfindungen ist bevorzugt, wenn der erfindungsgemäße Container (bevorzugt ein Metallcontainer) als Innenmaße eine Länge von 11,340 m, eine Breite von 2,658 m und eine Höhe von 2,710 m aufweist.

Die Sterilisationskammer bei der Ein-Container-Variante hat vorzugsweise ein Innenvolumen von mindestens 5,0 m³, weiter bevorzugt von mindestens 10,0 m³, noch weiter bevorzugt von mindestens 15,0 m³, noch weiter bevorzugt von mindestens 20 m³, weiter bevorzugt von mindestens 25 m³, noch weiter bevorzugt von mindestens 30,0 m³, weiter bevorzugt von mindestens 35,0 m³, weiter bevorzugt von mindestens 40,0 m³ und weiter bevorzugt von mindestens 45,0 m³. Die vorgenannten Werte gelten vorzugsweise für einen "40-ft-Container HC" und einen "45-ft-Container PW" und sind bei einem "20-ft-Container" entsprechend zu halbieren.

Anders ausgedrückt hat die Sterilisationskammer bei der Ein-Container-Variante vorzugsweise ein Innenvolumen von mindestens 5% des Innenvolumens des Metallcontainers, weiter bevorzugt von mindestens 7% des Innenvolumens des Metallcontainers, weiter bevorzugt von mindestens 9% des Innenvolumens des Metallcontainers, weiter bevorzugt von mindestens 10% des Innenvolumens des Metallcontainers, weiter bevorzugt von mindestens 12% des Innenvolumens des Metallcontainers, weiter bevorzugt von mindestens 15% des Innenvolumens des Metallcontainers, weiter bevorzugt von mindestens 18% des Innenvolumens des Metallcontainers, weiter bevorzugt von mindestens 20% des Innenvolumens des Metallcontainers, weiter bevorzugt von mindestens 25% des Innenvolumens des Metallcontainers, weiter bevorzugt von mindestens 30% des Innenvolumens des Metallcontainers, weiter bevorzugt von mindestens 35% des Innenvolumens des Metallcontainers weiter bevorzugt von mindestens 40% des Innenvolumens des Metallcontainers.

Die Sterilisationskammer bei der Zwei-Container-Variante oder einer Mehr-Container-Variante hat vorzugsweise ein Innenvolumen von mindestens, weiter bevorzugt von mindestens 30,0 m³, weiter bevorzugt von mindestens 32,0 m³, weiter bevorzugt von mindestens 35,0 m³, weiter bevorzugt von mindestens 38,0 m³, weiter bevorzugt von mindestens 40,0 m³, weiter bevorzugt von mindestens 45,0 m³, weiter bevorzugt von mindestens 50,0 m³, weiter bevorzugt von mindestens 55,0 m³, weiter bevorzugt von mindestens 60,0 m³. Die vorgenannten Werte gelten vorzugsweise für einen "40-ft-Container HC" und einen "45-ft-Container PW" und sind bei einem "20-ft-Container" entsprechend zu halbieren.

Anders ausgedrückt hat die Sterilisationskammer bei der Zwei-Container-Variante oder einer Mehr-Container-Variante vorzugsweise ein Innenvolumen von mindestens 35% des Innenvolumens des Metallcontainers, worin sich die Sterilisationskammer befindet, weiter bevorzugt von mindestens 38%, weiter bevorzugt von mindestens 40%, weiter bevorzugt von mindestens 42%, weiter bevorzugt von mindestens 45%, weiter bevorzugt von mindestens 50%, weiter bevorzugt von mindestens 55%, weiter bevorzugt von mindestens 60%, weiter bevorzugt von mindestens 65%, weiter bevorzugt von mindestens 70% des Innenvolumens des Metallcontainers, worin sich die Sterilisationskammer befindet.

Gemäß einer weiteren Ausführungsform betrifft die vorliegende Erfindung ebenfalls eine begehbare stationäre Ethylenoxid-Sterilisationsanlage, wobei die Sterilisationskammer bei der Ein-Container-Variante ein Innenvolumen von mindestens 5% des Innenvolumens des Metallcontainers hat oder wobei die Sterilisationskammer bei der Zwei-Container-Variante ein Innenvolumen von mindestens 35% des Innenvolumens des Metallcontainers hat, worin sich die Sterilisationskammer befindet.

Das Gesamtgewicht eines Containers als Teil der erfindungsgemäßen Sterilisationsanalage ist dann natürlich nicht nur von den Kennwerten des Containers abhängig, sondern auch davon, welche Komponenten in dem Container installiert sind.

Wenn es aufgrund der Abmessung der Sterilisationskammer und/oder der weiteren Bauteile der Sterilisationsanlage notwendig sein sollte, die Containergröße zu verändern, so kann der Container von den normierten Abmessungen abweichen. Dies kann beispielsweise in einer Zwei-Container-Variante der erfindungsgemäßen Sterilisationsanlage für den Container mit der Sterilisationskammer gelten. Die Abmessungen für einen solchen nicht-normierten Container mit Sterilisationskammer umfassend eine Sterilisationskammer für 16 Paletten (eine "16-Palettensterilisationskammer") können zum Beispiel 12,88m x 2,75m x 3,29m sein. Das Gewicht eines nicht-normierten Containers, welcher eine 16-Palettensterilisationskammer beinhaltet, liegt zwischen 20 t und 30 t, insbesondere zwischen 25 t und 27 t. Wenn die normierten Abmessungen nicht verwendet werden, kann es in bestimmten Ausführungsformen notwendig sein, dass Bauteile am Container für den Transport abmontiert werden, so dass die Transportform des Containers der Norm entspricht. Hier ist es dann bevorzugt, wenn die betreffenden Bauteile für den Transport in dem Container "zwischengelagert" werden können. Dies gilt insbesondere für Rolltore und deren Verkleidung oder Lüftungsanlagen, welche an einem normierten Container außen angebracht sind.

Besonders bevorzugt wird jedoch, wenn die vormontierte Sterilisationsanlage in einem oder mehreren normierten Containern installiert ist und die normierten Maße auch im vormontierten Zustand nicht überschreitet.

Diese normierten Container haben aufgrund der Normierung den großen Vorteil, dass die (nach Installation am Bestimmungsort stationäre) Ethylenoxid-Sterilisationsanlage in den normierten Containern vollständig eingebaut werden und in diesem Zustand per LKW, Zug oder Schiff mit geringem Aufwand transportiert werden kann. Vorzugsweise werden für die erfindungsgemäße Ethylenoxid-Sterilisationsanlage Container mit so genannten ISO-Ecken verwendet, die eine einfache Verbindung mit üblichen Transportsystemen (Containerbrücken, LKW-Tieflader, Kräne, usw.) oder auch mit anderen Containern ermöglicht und somit Transport und Lagerung vereinfacht und zudem sicherer macht. Am Bestimmungsort angekommen, brauchen dann die normierten Container nur noch mit den Außenleitungen verbunden zu werden, ohne dass die Einzelkomponenten der stationären Ethylenoxid-Sterilisationsanlage jeweils einer Installation bedürfen. Dies ist vor allem ein enormer Vorteil, wenn derartige stationäre Ethylenoxid-Sterilisationsanlagen in Schwellenländer wie beispielsweise Indien, Südamerika oder einigen asiatischen Staaten wie z.B. Indonesien, Malaysia, Philippinen, Vietnam, Thailand oder der gleichen geliefert werden, wo jede Installation vor Ort aufgrund des Fachkräftemangels die Anwesenheit ausländischer Mechaniker und Techniker bedarf.

Unter dem Begriff "stationäre" Ethylenoxid-Sterilisationsanlage soll verstanden werden, dass diese aufgrund Ihrer Dimensionen und ihres Gewichtes nicht von einzelnen Personen oder einer Gruppe von Personen zwischen 2 und 10 Personen von Hand transportiert werden kann. Dies steht im Gegensatz zu den tragbaren Sterilisationsanlagen aus dem Stand der Technik.

Dieser Begriff "stationär" schließt natürlich nicht aus, dass vor der Endinstallation am Bestimmungsort die in dem mindestens einen Container und vorzugsweise in dem mindestens einen Seecontainer montierte Ethylenoxid-Sterilisationsanlage transportiert werden kann, aber eben nur unter Zuhilfenahme von schweren Maschinen, die für derart hohe Traglasten ausgerichtet sind, wie z.B. Kränen. Genau in dieser Kombination der Merkmale liegt der große Vorteil der erfindungsgemäßen stationären Ethylenoxid-Sterilisationsanlage. Zum einen ist die Ethylenoxid-Sterilisationsanlage bis auf die Außenanschlüsse in dem mindestens einen Container und vorzugsweise in dem mindestens einen Seecontainer vollständig vormontiert und zum anderen befindet sich die erfindungsgemäße Ethylenoxid-Sterilisationsanlage in einem Gehäuse, nämlich mindestens einem Metallcontainer und vorzugsweise mindestens einem Seecontainer, so dass ein Transport auch über weite Strecken unmittelbar, einfach und kostengünstig erfolgen kann. Am Bestimmungsort angekommen, muss dann die stationäre Ethylenoxid-Sterilisationsanlage nicht noch aufwändig zusammengebaut werden, sondern braucht nur noch mit den Rohrleitungen von außen, d.h. den Außenanschlüssen verbunden zu werden und ist betriebsbereit. Somit ist die Vorrichtung für die Auslieferung der stationären Ethylenoxid-Sterilisationsanlage identisch mit dem Gehäuse für die stationäre Ethylenoxid-Sterilisationsanlage, weil der Metallcontainer und vorzugsweise der normierte Seecontainer beide Aufgaben übernimmt.

Daher ist eine bevorzugte Ausführungsform der vorliegenden Erfindung, dass sich zumindest Sterilisationskammer, Vakuumpumpe, Ethylenoxidverdampfer und Ethylenoxidtank in einem normierten Metallcontainer und vorzugsweise in einem normierten Seecontainer befinden.

Für den Fall, dass eine möglichst große Sterilisationskammer gewünscht wird, betrifft eine weitere Ausführungsform der vorliegenden Erfindung eine stationäre Ethylenoxid-Sterilisationsanlage, welche in zwei normierten Metallcontainern und vorzugsweise zwei normierten Seecontainern untergebracht ist. Bei dieser Ausführungsform, die zum Teil auch als Zwei-Container-Variante bezeichnet wird, sind in einem (ersten) Metallcontainer zumindest Vakuumpumpe, Ethylenoxidverdampfer und Ethylenoxidtank vorgesehen, und die Sterilisationskammer wird in einem (zweiten) Metallcontainer untergebracht, so dass der maximale Raum, nämlich der Raum eines Metallcontainers und vorzugsweise eines normierten Seecontainers, für die Sterilisationskammer zur Verfügung steht. Zusätzlich können in diesem ersten Metallcontainer auch noch die Befeuchtungsanlage, die Lüftungsanlage, die Kammerheizung und/oder die Abgasreinigungsanlage enthalten sein.

Die vorliegende Erfindung ist somit ebenfalls gerichtet auf eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage und eine Sterilisationskammer, wobei die Vakuumpumpe, der Ethylenoxidverdampfer und der Ethylenoxidtank in einem ersten Metallcontainer und die Sterilisationskammer in einem zweiten Metallcontainer untergebracht sind.

Sollte damit die Größe der Sterilisationskammer noch immer nicht ausreichen, so kann entweder von der normierten Größe für den Container der Sterilisationskammer abgewichen werden, oder es kann in einer weiteren bevorzugten Ausführungsform der erfindungsgemäßen stationären Ethylenoxid-Sterilisationsanlage vorgesehen werden, dass zumindest Vakuumpumpe, Ethylenoxidverdampfer und Ethylenoxidtank in einem ersten normierten Container (vorzugsweise Metallcontainer) untergebracht sind und an diesen ersten Container (vorzugsweise Metallcontainer) ein nicht normierter Container oder zwei oder mehr normierte Metallcontainer angeschlossen werden können, worin jeweils Sterilisationskammern vorgesehen sind, so dass der für die Sterilisation zur Verfügung stehende Raum durch einen dritten Metallcontainer verdoppelt, durch einen vierten verdreifacht und durch einen (X-1)-Metallcontainer X-fach vergrößert werden kann.

Da somit innerhalb des Metallcontainers bzw. der Metallcontainer der explosionsgefährdete Raum ist, erfolgt die Steuerung der stationären Ethylenoxid-Sterilisationsanlage vorzugsweise von außerhalb des Metallcontainers. Die Steuerung oder Steuervorrichtung für die stationäre Ethylenoxid-Sterilisationsanlage ist daher vorzugsweise außerhalb des mindestens einen Metallcontainers oder von außen am Metallcontainer angebracht. Hierbei ist bevorzugt, wenn die Steuerung derart in eine Vertiefung bzw. Aussparung im Containermantel eingelassen ist, dass sie zwar von außen für das Bedienpersonal zugänglich ist, aber nicht über den Containermantel hinausragt. Zudem kann die Steuerung durch eine Klappe oder Tür verschlossen werden, wodurch die Steuerung beim Transport und nach dem Aufstellen der Anlage nicht den Widrigkeiten der Umwelt ausgesetzt ist. Ist die Steuerung hingegen im Metallcontainer untergebracht, so befindet sich diese vorzugsweise in einem innerhalb des Metallcontainers räumlich abgetrennten Bereich (auch als Kontrollraum oder Steuerungsraum bezeichnet). Werden zwei oder mehr normierte Metallcontainer verwendet, so befinden sich im ersten normierten Metallcontainer zumindest Vakuumpumpe, Ethylenoxidverdampfer und Ethylenoxidtank und optional Befeuchtungsanlage, Steuerung (auch als Steuervorrichtung bezeichnet) und Kammerheizung. In dem oder den weiteren normierten Metallcontainers befinden sich Sterilisationskammern. Auch außerhalb des Metallcontainers oder der Metallcontainer kann sich die Abgasreinigungsanlage befinden.

Gemäß einer weiteren Ausführungsform betrifft die vorliegende Erfindung somit ebenfalls eine begehbare stationäre Ethylenoxid-Sterilisationsanlage, wobei die Steuerung (der begehbaren stationären Ethylenoxid-Sterilisationsanlage) außerhalb des mindestens einen Metallcontainers oder von außen an dem mindestens einen Metallcontainer angebracht ist.

Gemäß noch einer weiteren Ausführungsform betrifft die vorliegende Erfindung ebenfalls eine begehbare stationäre Ethylenoxid-Sterilisationsanlage, wobei die Steuerung (der begehbaren stationären Ethylenoxid-Sterilisationsanlage) in einem räumlich abgetrennten Bereich innerhalb des mindestens einen Metallcontainers angebracht ist.

Die "Kammerheizung" dient dazu, die Sterilisationskammer während des Sterilisationsvorganges auf die gewünschte Betriebstemperatur aufzuheizen. Dem Fachmann sind aus dem Stand der Technik zahlreiche mögliche Ausführungsformen einer solchen Kammerheizung für Sterilisationsanlagen bekannt. Mögliche Ausführungsformen der Kammerheizung in der vorliegenden Erfindung umfassen aber sind nicht beschränkt auf: Warmwassersysteme, Heißluft-basierte Systeme sowie Systeme mit anderen zur Wärmleitung befähigten Medien (z.B. Ölen).

In einer weiter bevorzugten Ausführungsform der vorliegenden Erfindung befindet sich jedoch auch die Abgasreinigungsanlage innerhalb des oder der Metallcontainer der Ethylenoxid-Sterilisationsanlage.

Bei der "Abgasreinigungsanlage" bzw. der "EO-Entsorgung" handelt es sich um eine Vorrichtung, welche dazu dient, die im Sterilisationsprozess anfallenden toxischen Gasgemische, die Ethylenoxid enthalten, zu entgiften, so dass diese ohne Gefährdung der Umwelt abgelassen werden können. Gemäß einigen Ausführungsformen ist die Abgasreinigungsanlage an die Vakuumpumpe angeschlossen, welche in Verbindung mit der Sterilisationskammer steht. Nach der Sterilisation wird folglich das Ethylenoxid mittels der Vakuumpumpe aus der Sterilisationskammer abgezogen und in die Abgasreinigungsanlage weitergeleitet. Gemäß einigen Ausführungsformen ist es möglich, dass sich die Abgasreinigungsanlage außerhalb des Metallcontainers der erfindungsgemäßen Sterilisationslange befindet. Es ist jedoch besonders bevorzugt, wenn sich die Abgasreinigungsanlage innerhalb des Metallcontainers der erfindungsgemäßen Sterilisationslange befindet. In diesem Fall ist es möglich, dass die Abgasreinigungsanlage die gereinigten und vom Ethylenoxid befreiten Abgase nach draußen leitet. Es ist allerdings auch denkbar, dass der Auslass für die gereinigten Abgase an der Abgasreinigungsanlage mit der Lüftungsanlage des Metallcontainers verbunden ist und somit die gereinigten Abgase über die Lüftungsanlage außerhalb des Containers befördert werden. Ausführungsformen der Abgasreinigungsanlage zur Entsorgung des Ethylenoxids umfassen eine Vorrichtung zur thermischen Abgasreinigung (Fackel), eine Vorrichtung zur katalytischen Verbrennung, einen chemischen Wäscher oder Filteranlagen.

Die vorliegende Erfindung ist somit ebenfalls gerichtet auf eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind, wobei die begehbare stationäre Ethylenoxid-Sterilisationsanlage des Weiteren eine Abgasreinigungsanlage und eine Kammerheizung umfasst, wobei sich Abgasreinigungsanlage und Kammerheizung innerhalb des mindestens einen Metallcontainers befinden.

Die vorliegende Erfindung ist somit ebenfalls gerichtet auf eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage, eine Sterilisationskammer, eine Abgasreinigungsanlage und eine Kammerheizung, dadurch gekennzeichnet, dass die Sterilisationskammer, die Vakuumpumpe, der Ethylenoxidverdampfer, der Ethylenoxidtank die Abgasreinigungsanlage und die Kammerheizung in mindestens einem Metallcontainer untergebracht sind.

Die vorliegende Erfindung ist somit ebenfalls gerichtet auf eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage, eine Sterilisationskammer, eine Abgasreinigungsanlage und eine Kammerheizung, dadurch gekennzeichnet, dass die Sterilisationskammer, die Vakuumpumpe, der Ethylenoxidverdampfer, der Ethylenoxidtank die Abgasreinigungsanlage und die Kammerheizung in mindestens einem Metallcontainer untergebracht sind, und des Weiteren umfassend Rohrleitungen zwischen Sterilisationskammer und Vakuumpumpe, zwischen Vakuumpumpe und Abgasreinigungsanlage, zwischen Sterilisationskammer und Ethylenoxidverdampfer, zwischen Ethylenoxidverdampfer und Ethylenoxidtank sowie zwischen Sterilisationskammer und Befeuchtungsanlage.

Die Unterbringung der explosionsgefährdeten Bauteile der stationären Ethylenoxid-Sterilisationsanlage in einem Metallcontainer (bevorzugt in einem normierten Metallcontainer) hat ferner den Vorteil, dass die Anlage in diesem Metallcontainer vormontiert werden kann, der komplette Metallcontainer vom Hersteller zum Kunden ausgeliefert und dort die Endmontage deutlich einfacher vorgenommen werden kann, weil nur noch die entsprechenden Zu- und Ableitungen zu dem oder den Metallcontainer(n) geschaffen werden müssen aber die Sterilisationsanlage an sich bereits fertig im Metallcontainer montiert ist. Damit wird die Installation vor Ort vereinfacht. Zusätzliche Verrohrungsarbeiten zwischen den explosionsgefährdeten Bauteilen der Sterilisationsanlage vor Ort sind nicht mehr notwendig. Zudem werden die prozessführenden Rohrleitungen kürzer, da sich alle relevanten Bauteile in mindestens einem Metallcontainer nahe beieinander befinden und nicht mehr räumlich getrennt sind. Um die Installation vor Ort so einfach wie möglich zu gestalten, können auch die Steuerung für die EO-Sterilisationsanlage, sowie die Lüftungsanlage und die Befeuchtungsanlage innerhalb des Metallcontainers angeordnet sein, worin sich Vakuumpumpe, Ethylenoxidverdampfer und Ethylenoxidtank befinden und vorzugsweise durch eine räumliche Abtrennung von den anderen Komponenten der EO-Sterilisationsanlage separiert werden. In bestimmten Ausführungsformen der Ein-Container-Variante ist die räumliche Abtrennung innerhalb des einen Metallcontainers, die die Sterilisationskammer, die Lüftungsanlage und die Befeuchtungsanlage räumlich von den anderen Komponenten der Ethylenoxid-Sterilisationsanlage trennt, nicht zwingend explosionssicher gestaltet. In anderen, bevorzugten Ausführungsformen ist jedoch bevorzugt, wenn auch diese räumliche Abtrennung explosionssicher ausgestaltet ist.

Der Ethylenoxidtank ist vorzugsweise eine Ethylenoxid-Gasflasche (EO-Gasflasche) oder ein Ethylenoxid-Gebinde (EO-Gebinde) Bevorzugt ist eine Gasflasche, welche in Abmessungen und Befüllung auf die jeweils konkret verwendete bzw. verbaute Sterilisationskammer abgestimmt ist und eine ausreichende Menge an Ethylenoxid enthält, um die für die Sterilisation notwendige Ethylenoxid-Konzentration in der Sterilisationskammer von 400-800 g/m³ zu erzeugen. Für eine Sterilisationskammer für 16 Paletten ("16-Paletten-Sterilisationskammer") liegt die notwendige Menge an Ethylenoxid beispielsweise bei 15-30 kg, insbesondere bei 20-25 kg. Es können sowohl eine, als auch mehrere Ethylenoxid-Gasflaschen angeschlossen werden. Hierbei kann der Flascheninhalt einer einzelnen Ethylenoxid-Gasflasche genug Ethylenoxid für mehrere Sterilisationszyklen beinhalten, bevorzugt wird jedoch eine einmalige Füllung, d.h. für jeden Sterilisationszyklus wird der Inhalt einer ganzen Ethylenoxid-Gasflasche verwendet.

Gängige Konfektionen für Gasflaschen, die im Rahmen der vorliegenden Erfindung verwendet werden können, umfassen Gasflaschen mit einem Volumen von 10 L, 12 L, 20 L, 27 L, 33 L, 40 L, 47 L, 50 L oder 75 L. Für die Lagerung von Ethylenoxid werden in der Regel Gasflaschen aus Stahl bzw. aus Edelstahl verwendet.

Ein weiterer Vorteil liegt darin, dass Störfälle (insbesondere Leckagen) durch den mindestens einen Metallcontainer räumlich begrenzt werden. Durch die vorzugsweise außen am Metallcontainer angebrachte Steuerung, welche den Zustand der Sterilisationsanlage samt der explosionsgefährdeten Bauteile anzeigt, wird die Mitarbeitergefährdung minimiert. Ein Zugang zum mindestens einen Metallcontainer wird beispielsweise nur erlaubt, wenn keine unmittelbare Gefahr besteht. Die Anordnung der explosionsgefährdeten Bauteile der stationären Ethylenoxid-Sterilisationsanlage hat zudem den Vorteil, dass im laufenden Betrieb eine Überwachung von mehreren Räumen (EO-Waagenraum, EO-Verdampferraum, Sterilisationsbereich) nicht mehr notwendig ist. Die Überwachung des Containerinnenraums oder der Containerinnenräume bzw. der Abluft dieser Räume genügt vollkommen und ist vorzugweise in die Steuerung der Ethylenoxid-Sterilisationsanlage integriert. Zusätzlich kann die Steuerung der Ethylenoxid-Sterilisationsanlage die Überwachung der Zugangsbereiche (Türen) mit übernehmen und einen Start eines Sterilisationszyklus nur dann freigeben, wenn alle Türen geschlossen sind und die Lüftungsanlage in Betrieb ist. Diese Maßnahme garantiert kontrollierte Umgebungsbedingungen für die Sterilisationsanlage.

Folglich ist die vorliegende Erfindung gemäß einer weiteren Ausführungsform ebenfalls gerichtet auf eine begehbare stationäre Ethylenoxid-Sterilisationsanlage, wobei die begehbare stationäre Ethylenoxid-Sterilisationsanlage zusätzlich Ethylenoxid-Sensoren zur Überwachung der Ethylenoxid-Konzentration in der begehbaren stationären Ethylenoxid-Sterilisationsanlage sowie ein System zur Kontrolle des Öffnungszustandes von Zugängen zur Sterilisationskammer umfasst, und wobei die Ethylenoxid-Sensoren und das System zur Kontrolle des Öffnungszustandes von Zugängen zur Sterilisationskammer an die Steuerung angeschlossen sind.

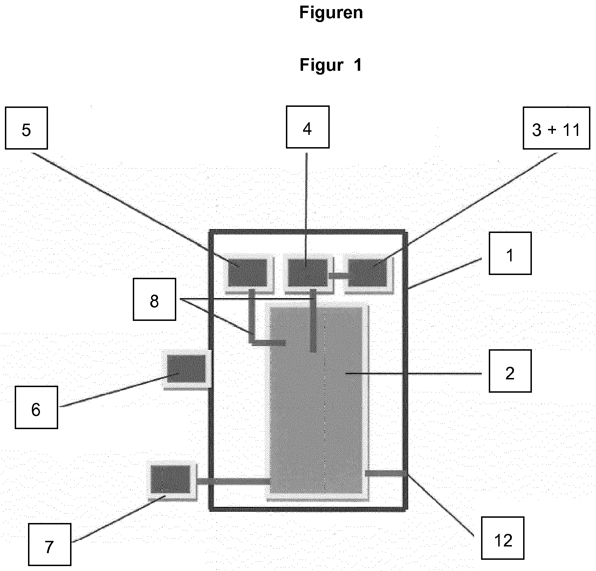

Figur 1 zeigt stark vereinfacht einen schematischen Aufbau einer erfindungsgemäßen stationären Ethylenoxid-Sterilisationsanlage umfassend eine Vakuumpumpe (5), einen Ethylenoxidverdampfer (4), einen Ethylenoxidtank (3), eine Steuerung oder Steuervorrichtung (6), eine Befeuchtungsanlage (7) in Form eines Dampfgenerators (7a), erforderliche Rohrleitungen (8) und eine Sterilisationskammer (2). Die explosionsgefährdeten Bauteile umfassend die Sterilisationskammer (2), die Vakuumpumpe (5), den Ethylenoxidverdampfer (4) und den Ethylenoxidtank (3) sind in dem Metallcontainer (1) bzw. Seecontainer untergebracht. Steuerung oder Steuervorrichtung (6) für die Sterilisationsanlage sowie Befeuchtungsanlage (7) sind außerhalb des Metallcontainers (1) vorgesehen.

Zwingend mittels Rohrleitungen (8) innerhalb des Metallcontainers (1) verbunden sind der Ethylenoxidtank (3) mit dem Ethylenoxidverdampfer (4) und dieser wiederum mit der Sterilisationskammer (2). Die Sterilisationskammer (2) ist des Weiteren über Rohrleitungen (8) innerhalb des Metallcontainers (1) mit mindestens einer Vakuumpumpe (5) verbunden. Wie in Figur 1 gezeigt, kann sich der mindestens eine Dampfgenerator (7a) bzw. die mindestens eine Befeuchtungsanlage (7) außerhalb des Metallcontainers (1) befinden, kann aber auch innerhalb des Metallcontainers (1) angeordnet sein. Auch zwischen Befeuchtungsanlage (7) und Sterilisationskammer (2) verlaufen entsprechende Rohrleitungen. Die Befeuchtungsanlage (7) ist vorzugsweise ein Dampfgenerator (7a). Befindet sich der Dampfgenerator (7a) außerhalb des Metallcontainers (1), so führt die Rohrleitung zwischen Dampfgenerator (7a) und Sterilisationskammer (2) durch den Containermantel hindurch. Des Weiteren gibt es eine Leitung für die Kammerheizung, diese kann sowohl in dem Metallcontainer (1), als auch außerhalb angeordnet sein. Ist diese außerhalb angeordnet, ist die Rohrleitung zwischen Sterilisationskammer (2) und Kammerheizung (z.B. ein Warmwassersystem) eine zweite Rohrleitung, die durch den Containermantel hindurchführt. Als dritte Leitung, welche durch den Containermantel hindurchführt, ist die Leitung von Vakuumpumpe (5) zur Abgasreinigungsanlage (14). Die Abgasreinigungsanlage (14) ist vorzugsweise außerhalb des Gebäudes angeordnet, kann aber auch innerhalb des Metallcontainers (1) angeordnet werden.

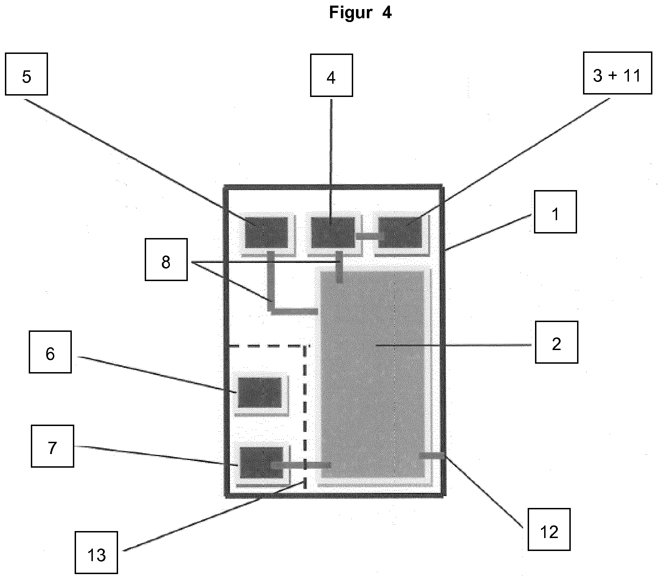

Figur 4 zeigt einen schematischen Aufbau einer erfindungsgemäßen stationären Ethylenoxid-Sterilisationsanlage in einer möglichen Ein-Container-Variante umfassend eine Vakuumpumpe (5), einen Ethylenoxidverdampfer (4), einen Ethylenoxidtank (3), eine Steuerung oder Steuervorrichtung (6), eine Befeuchtungsanlage (7) in Form eines Dampfgenerators (7a), erforderliche Rohrleitungen (8) und eine Sterilisationskammer (2). Die explosionsgefährdeten Bauteile umfassend die Sterilisationskammer (2), die Vakuumpumpe (5), den Ethylenoxidverdampfer (4) und den Ethylenoxidtank (3) sind in dem Metallcontainer (1) bzw. Seecontainer untergebracht. Steuerung oder Steuervorrichtung (6) für die Sterilisationsanlage sowie Befeuchtungsanlage (7) sind ebenfalls innerhalb des Metallcontainers (1) vorgesehen und vorzugsweise in einem räumlich getrennten Bereich innerhalb des Metallcontainers (1).

Zwingend mittels Rohrleitungen (8) innerhalb des Metallcontainers (1) verbunden sind der Ethylenoxidtank (3) mit dem Ethylenoxidverdampfer (4) und dieser wiederum mit der Sterilisationskammer (2). Die Sterilisationskammer (2) ist des Weiteren über Rohrleitungen (8) innerhalb des Metallcontainers (1) mit mindestens einer Vakuumpumpe (5) verbunden. Wie in Figur 4 gezeigt, kann sich der mindestens eine Dampfgenerator (7a) bzw. die mindestens eine Befeuchtungsanlage (7) auch innerhalb des Metallcontainers (1) befinden. Auch zwischen Befeuchtungsanlage (7) und Sterilisationskammer (2) verlaufen entsprechende Rohrleitungen innerhalb des Metallcontainers (1). Die Befeuchtungsanlage (7) ist vorzugsweise ein Dampfgenerator (7a).

Auch die Kammerheizung kann in dem Metallcontainer (1) untergebracht sein. Dann verläuft eine Leitung zwischen Kammerheizung und Sterilisationskammer (2) innerhalb des Metallcontainers (1). Eine Leitung, welche durch den Containermantel hindurchführt, ist die Leitung von Vakuumpumpe (5) zur Abgasreinigungsanlage (14). Die Abgasreinigungsanlage (14) ist vorzugsweise außerhalb des Gebäudes angeordnet, kann aber auch innerhalb des Metallcontainers (1) angeordnet werden. Neben der bereits angesprochenen Ein-Container-Variante der erfindungsgemäßen Ethylenoxid-Sterilisationsanlage, werden auch Zwei-Container- oder sogar Mehr-Container-Varianten erfindungsgemäß bevorzugt. Deren Vorteil geht weit über die bereits diskutierte Vergrößerung des zur Verfügung stehenden Sterilisationskammervolumens hinaus und liegt besonders in dem modularen Charakter einer solchen Anlage.

Somit betrifft die vorliegende Erfindung ebenfalls eine begehbare stationäre Ethylenoxid-Sterilisationsanlage umfassend eine Vakuumpumpe, einen Ethylenoxidverdampfer, einen Ethylenoxidtank, eine Steuerung, eine Befeuchtungsanlage und eine Sterilisationskammer, dadurch gekennzeichnet, dass die Sterilisationskammer, die Vakuumpumpe, der Ethylenoxidverdampfer und der Ethylenoxidtank in mindestens zwei Metallcontainern untergebracht sind, wobei die begehbare stationäre Ethylenoxid-Sterilisationsanlage ein modulares System darstellt. Der Begriff "modulares System" wird hierin gleichbedeutend mit dem Begriff "Modulsystem" verwendet werden.

Somit betrifft die vorliegende Erfindung ebenfalls eine begehbare stationäre Ethylenoxid-Sterilisationsanlage in Gestalt eines modularen Systems umfassend einen ersten Metallcontainer enthaltend eine Vakuumpumpe, einen Ethylenoxidverdampfer, einen Ethylenoxidtank, eine Steuerung und eine Befeuchtungsanlage (und optional eine Lüftungsanlage, eine Kammerheizung und/oder eine Abgasreinigungsanlage) sowie mindestens einen weiteren Metallcontainer enthaltend eine Sterilisationskammer, wobei der erste Metallcontainer verbindbar mit in der Größe variablen weiteren Metallcontainern enthaltend eine Sterilisationskammer ist.

Geht man beispielsweise von der bereits oben beschriebenen Ausführungsform einer Zwei-Container-Variante der vorliegenden Erfindung aus, bei der in einem ersten Metallcontainer zumindest eine Vakuumpumpe, ein Ethylenoxidverdampfer, ein Ethylenoxidtank, eine Steuerung und eine Befeuchtungsanlage (und je nach Ausführungsform optional zusätzlich eine Lüftungsanlage, eine Kammerheizung und eine Abgasreinigungsanlage) untergebracht sind, während in einem zweiten Container eine Sterilisationskammer untergebracht ist, so ist es in einer derartigen Ausführungsform möglich, dass der erste Metallcontainer (hier auch als Steuerungscontainer bezeichnet) mit einem zweiten Metallcontainer (hier auch als Sterilisationscontainer bezeichnet), der die Sterilisationskammer enthält, unterschiedlichster Größe kombiniert werden kann. Je nach Bedarf des Anwenders könnte z.B. der Steuerungscontainer mit einem Sterilisationscontainer in Form eines "20-ft-Containers" oder auch mit einem Sterilisationscontainer in Form eines "40-ft-Containers" kombiniert werden. Dies ist für den Anwender von enormem Vorteil. Er kann sich nicht nur seine Sterilisationsanlage bedarfsgerecht liefern lassen, sondern er kann auch bei einer Änderung seines Bedarfes die Anlage durch Austauschen z.B. des Sterilisationscontainers an die aktuellen Gegebenheiten anpassen, ohne gleich die komplette Anlage austauschen zu müssen. Bei einem gesteigerten Bedarf an Sterilprodukten kann z.B. ein kleiner Sterilisationscontainer durch einen größeren Sterilisationscontainer ausgetauscht werden, während der Steuerungscontainer beibehalten wird. Bei einem Rückgang des Bedarfes kann hingegen ein großer Sterilisationscontainer durch einen kleineren Sterilisationscontainer ausgetauscht werden, während der Steuerungscontainer beibehalten wird, so dass ein unnötiger Materialaufwand (z.B. durch erhöhten Ethylenoxidverbrauch bei einer zu großen Kammer) und damit unnötige Kosten vermieden werden können. Dementsprechend ist es bevorzugt, wenn die Verbindungen zwischen dem Steuerungscontainer und dem Sterilisationscontainer reversibel sind, d.h. beispielsweise nicht geschweißt sondern geschraubt sind.

Unter dem bereits beschriebenen Aufwand (schwere Maschinen zum Transport) ist es so verhältnismäßig einfach, z.B. einzelne Container zwischen verschiedenen Standorten auszutauschen, ohne hierbei gleich neue Anlagen erwerben zu müssen.

Der Begriff "Steuerungscontainer", wie hierin verwendet, bezeichnet in einer Zwei-Container- oder Mehr-Container Variante der vorliegenden Erfindung den Container, der zumindest die Steuerung der Ethylenoxid-Sterilisationsanlage umfasst, und ist ausschließlich auf Ausführungsformen anzuwenden, bei denen die Sterilisationskammer sowie die Steuerung der Ethylenoxid-Sterilisationsanlage in unterschiedlichen Containern untergebracht sind. Die Steuerrung kann hierbei auch an dem Steuerungscontainer angebracht sein und muss sich nicht zwangsläufig in diesem befinden.

Der Begriff "Sterilisationscontainer", wie hierin verwendet, bezeichnet in einer Zwei-Container- oder Mehr-Container Variante der vorliegenden Erfindung den Container, der zumindest die Sterilisationskammer der Ethylenoxid-Sterilisationsanlage enthält, und ist ausschließlich auf Ausführungsformen anzuwenden, bei denen die Sterilisationskammer sowie die Steuerung der Ethylenoxid-Sterilisationsanlage in unterschiedlichen Containern untergebracht sind. Die Steuerrung kann hierbei auch an dem Steuerungscontainer angebracht sein und muss sich nicht zwangsläufig in diesem befinden.

Bei der Kombination unterschiedlicher Container in einem modularen System ist es natürlich wichtig, dass die zur Verfügung stehenden Anschlüsse zwischen den Containern (z.B. von dem Ethylenoxidverdampfer im Steuerungscontainer zu der Sterilisationskammer im Sterilisationscontainer) derart ausgestaltet sind, dass sie zu allen angebotenen Container-Varianten eines Modulsystems kompatibel sind, also aufeinander bzw. zueinander passen.

Wie bereits oben beschrieben, ist es natürlich in einem derartigen modularen System auch möglich, einen Steuerungscontainer mit zwei oder sogar mehr Sterilisationscontainern (eventuell unterschiedlicher Größe) zu kombinieren. Bei einer Ethylenoxid-Sterilisationsanlage bestehend aus drei oder mehr Containern, wird hier auch von einer "Mehr-Container-Variante" der vorliegenden Erfindung gesprochen. Durch Hinzufügen eines weiteren Sterilisationscontainers an den bereits vorhandenen Sterilisationscontainer kann das zur Sterilisation in einer Sterilisationskammer zur Verfügung stehende Volumen vergrößert und den aktuellen Bedürfnissen angepasst werden. Ein Steuerungscontainer kann somit ohne umgerüstet oder ausgetauscht zu werden, eine variable Anzahl an Sterilisationscontainern steuern. Ein derart flexibler, modularer und kostengünstiger Aufbau einer begehbaren Sterilisationsanlage ist im Stand der Technik nicht bekannt und ist vor allem für die Industrie in Schwellenländern von großem Vorteil, welche beginnen, mit eigenen beschränkten Mitteln Medizinprodukte herzustellen und zu sterilisieren und nach einiger Zeit bereits an die Grenzen der Sterilisationsanlage stoßen. Das hierin beschriebene modulare System ermöglicht den unkomplizierten und kostengünstigen Ausbau einer bestehenden Zwei-Container-Sterilisationsanlage, ohne gleich die gesamte Anlage verschrotten und durch eine neue ersetzen zu müssen.

Hierzu sind mehrere technische Umsetzungen möglich, die durchaus auch miteinander kombiniert werden können. So kann z.B. bei einer modularen "Mehr-Container-Variante" der Steuerungscontainer Anschlüsse für mehrere Sterilisationscontainer besitzen. Diese können beispielsweise auf verschiedenen Seiten des Steuerungscontainers liegen. So wäre es z.B. möglich, dass in einer montierten Ethylenoxid-Sterilisationsanlage ein Steuerungscontainer mittig angeordnet ist, während sich auf seiner linken und rechten Seite jeweils ein Sterilisationscontainer befindet. Natürlich sind auch anderen Anordnungen möglich.

Es ist aber ebenso möglich, dass bei einer modularen "Mehr-Container-Variante" ein Sterilisationscontainer nicht nur mit dem Steuerungscontainer verbunden werden kann, sondern auch mit einem oder mehreren weiteren Sterilisationscontainer, so dass letztlich der Steuerungscontainer direkt nur mit einem Sterilisationscontainer verbunden ist, welcher aber zusammen mit den an ihn angeschlossenen weiteren Sterilisationscontainern eine Art Sterilisationscontainersystem oder -kontinuum darstellt. So wäre es z.B. möglich, dass in einer montierten Ethylenoxid-Sterilisationsanlage ein Steuerungscontainer links angeordnet ist, während sich auf seiner rechten Seite ein Sterilisationscontainer befindet, der wiederum auf seiner rechten Seite mit einem weiteren Sterilisationscontainer verbunden ist. Natürlich sind auch andere Anordnungen möglich.

Erfindungsgemäß ist es natürlich auch denkbar, dass diese beiden Modulvarianten miteinander kombiniert werden. So wäre es z.B. möglich, dass in einer montierten Ethylenoxid-Sterilisationsanlage ein Steuerungscontainer zentral angeordnet ist, während sich auf seiner linken Seite ein Sterilisationscontainer und auf seiner rechten Seite zwei miteinander verbundene Sterilisationscontainer befinden. Natürlich sind auch andere Anordnungen möglich.

Bei derartigen modularen "Mehr-Container-Varianten", bei denen Steuerungscontainer und/oder Sterilisationscontainer Anschlüsse für mehrere weitere Container aufweisen, ist es erfindungsgemäß bevorzugt, wenn diese Anschlüsse jeweils optional betrieben werden können. Das kann einerseits bedeuten, dass die Anschlüsse dicht verschließbar sind, so dass z.B. an einen Steuerungscontainer mit mehreren Anschlüssen bei Bedarf auch nur ein Anschluss verwendet werden kann, während die anderen (momentan) nicht verwendeten Anschlüsse dicht verschlossen bleiben.

Zudem ist bevorzugt, wenn bei derartigen modularen "Mehr-Container-Varianten", bei denen Steuerungscontainer und/oder Sterilisationscontainer Anschlüsse für mehrere weitere Container aufweisen, in den Leitungen Vorrichtungen zu einem elektronisch gesteuerten Verschluss der Leitung vorhanden sind (z.B. Ventile), die durch die Steuerung der Ethylenoxid-Sterilisationsanlage angesteuert werden können. Dies ermöglicht z.B. bei einen modularen System aus einem Steuerungscontainer und beispielsweise zwei Sterilisationscontainern, dass bei Bedarf trotz einer existierenden Verbindung nur einer der beiden Sterilisationscontainer verwendet werden kann.

In diesen modularen Systemen bedeutet natürlich der Austausch eines kleinen Sterilisationscontainers gegen einen größeren Sterilisationscontainer (mit größerer Sterilisationskammer), dass der Bedarf an Ethylenoxid pro Sterilisationszyklus steigt, aber auch, dass die geforderte Leistung von Vakuumpumpe, Befeuchtungsanlage, Lüftungsanlage, usw. höher ist als für einen kleineren Sterilisationscontainer. In diesen modularen Systemen ist daher bevorzugt, wenn in dem Steuerungscontainer die technischen Komponenten (z.B. Vakuumpumpe, Ethylenoxidverdampfer, Lüftungsanlage, Befeuchtungsanlage, Abgasreinigungsanlage) derart ausgelegt bzw. dimensioniert sind und ausreichend Stauraum und Anschlüsse für ausreichend große bzw. ausreichend viele Ethylenoxidtanks bzw. Ethylenoxidflaschen vorgesehen sind, dass unterschiedlich große Sterilisationskammern (bzw. mehrere miteinander verbundene Sterilisationskammern) betrieben werden können. Dies hat natürlich den Vorteil, dass die kontinuierlich notwendigen Wartungsarbeiten (vor allem das Austauschen entleerter Ethylenoxidtanks) vornehmlich auf einen Container, nämlich den Steuerungscontainer, beschränkt sind. Wird also ein System aufgebaut, bei dem die Sterilisationsanlage einen Sterilisationscontainer umfasst, welcher nicht die maximale Kapazität des Steuerungscontainers ausschöpft, so ist z.B. Ethylenoxid für mehr als einen Sterilisationsprozess vorhanden, während bei voller Ausschöpfung der Kapazität des Steuerungscontainers nur ausreichend Ethylenoxid für einen Sterilisationsprozess vorhanden ist.

Bei einer gegebenen Größe eines Steuerungscontainers sind der Variabilität bzw. der Ausdehnung des Sterilisationskammervolumens natürlich Grenzen gesetzt.

Eine weitere erfindungsgemäße Ausführungsform der Ethylenoxid-Sterilisationsanlage besteht daher darin, dass die Ethylenoxidversorgung (also Ethylenoxidtank, Ethylenoxidverdampfer und auch die Leitungen zwischen Ethylenoxidtank und Ethylenoxidverdampfer sowie zwischen Ethylenoxidverdampfer und Sterilisationskammer) ebenfalls in dem Sterilisationscontainer enthalten und an das Volumen von dessen Sterilisationskammer angepasst sind. Dies erhöht zwar die kontinuierlich notwendigen Wartungsarbeiten, da diese sich auf mehrere Container ausdehnen, ermöglicht aber eine größerer Variabilität, da jeder Sterilisationscontainer eine eigene (aber von der Steuerung im Steuerungscontainer kontrollierte) Ethylenoxidversorgung besitzt.

Noch eine weitere erfindungsgemäße Ausführungsform der Ethylenoxid-Sterilisationsanlage besteht darin, dass sowohl die Ethylenoxidversorgung (also Ethylenoxidtank, Ethylenoxidverdampfer und auch die Leitungen zwischen Ethylenoxidtank und Ethylenoxidverdampfer sowie zwischen Ethylenoxidverdampfer und Sterilisationskammer) als auch die Ethylenoxid-Entsorgung (also Vakuumpumpe und Abgasreinigungsanlage und auch die Leitungen zwischen Sterilisationskammer und Vakuumpumpe sowie zwischen Vakuumpumpe und Abgasreinigungsanlage) ebenfalls in dem Sterilisationscontainer enthalten und an das Volumen von dessen Sterilisationskammer angepasst sind. D.h. in diesem Fall würde(n) der bzw. die Sterilisationscontainer des modularen Systems alle explosionsgefährdeten Komponenten der erfindungsgemäßen Ethylenoxid-Sterilisationsanlage beinhalten, was neben der erhöhten Variabilität des Systems auch die Sicherheit des Bedienpersonals an der Steuerung erhöht.

Noch eine weitere erfindungsgemäße Ausführungsform der Ethylenoxid-Sterilisationsanlage besteht darin, dass sowohl die Ethylenoxidversorgung (also Ethylenoxidtank, Ethylenoxidverdampfer und auch die Leitungen zwischen Ethylenoxidtank und Ethylenoxidverdampfer sowie zwischen Ethylenoxidverdampfer und Sterilisationskammer) als auch die Ethylenoxid-Entsorgung (also Vakuumpumpe und Abgasreinigungsanlage und auch die Leitungen zwischen Sterilisationskammer und Vakuumpumpe sowie zwischen Vakuumpumpe und Abgasreinigungsanlage) und die Befeuchtungsanlage ebenfalls in dem Sterilisationscontainer enthalten und an das Volumen von dessen Sterilisationskammer angepasst sind.

Eine zusätzliche erfindungsgemäße Ausführungsform der Ethylenoxid-Sterilisationsanlage besteht darin, dass sowohl die Ethylenoxidversorgung (also Ethylenoxidtank, Ethylenoxidverdampfer und auch die Leitungen zwischen Ethylenoxidtank und Ethylenoxidverdampfer sowie zwischen Ethylenoxidverdampfer und Sterilisationskammer) als auch die Ethylenoxid-Entsorgung (also Vakuumpumpe und Abgasreinigungsanlage und auch die Leitungen zwischen Sterilisationskammer und Vakuumpumpe sowie zwischen Vakuumpumpe und Abgasreinigungsanlage) und die Befeuchtungsanlage und die Kammerheizung ebenfalls in dem Sterilisationscontainer enthalten und an das Volumen von dessen Sterilisationskammer angepasst sind.

Eine zusätzliche erfindungsgemäße Ausführungsform der Ethylenoxid-Sterilisationsanlage besteht darin, dass sowohl die Ethylenoxidversorgung (also Ethylenoxidtank, Ethylenoxidverdampfer und auch die Leitungen zwischen Ethylenoxidtank und Ethylenoxidverdampfer sowie zwischen Ethylenoxidverdampfer und Sterilisationskammer) als auch die Ethylenoxid-Entsorgung (also Vakuumpumpe und Abgasreinigungsanlage und auch die Leitungen zwischen Sterilisationskammer und Vakuumpumpe sowie zwischen Vakuumpumpe und Abgasreinigungsanlage) und die Befeuchtungsanlage, die Kammerheizung und die Lüftungsanlage ebenfalls in dem Sterilisationscontainer enthalten und an das Volumen von dessen Sterilisationskammer angepasst sind.

Bei einer besonders bevorzugten modularen Variante einer begehbaren Mehrcontainersterilisationsanlage sind die mindestens zwei Sterilisationscontainer in Reihe geschaltet, d.h. stehen hintereinander. Diese Anlage kann durch weitere in Reihe angeordnete Sterilisationscontainer erweitert werden. Ferner ist dabei bevorzugt, wenn die in Reihe angeordneten Sterilisationscontainer zusammen eine gemeinsame Sterilisationskammer bilden, d.h. sich die Sterilisationskammern der einzelnen Sterilisationscontainer zu einer einzigen Sterilisationskammer vereinen. Diese Anordnung ist insbesondere bevorzugt für die Sterilisation von größeren Stückzahlen desselben Produktes. Zudem ist dabei bevorzugt, wenn die in Reihe angeordneten Sterilisationscontainer von einer Seite beladen und von der gegenüberliegenden Seite entladen werden können. D.h. es ist bevorzugt, wenn der erste in Reihe angeordnete Sterilisationscontainer von der Vorderseite beladen und der letzte in Reihe geschaltete Sterilisationscontainer von seiner Hinterseite entladen werden kann. Dabei ist ferner bevorzugt, wenn die Paletten durch die in Reihe angeordneten Sterilisationscontainer auf Mitteln zur Beförderung bewegt werden können. Derartige Mittel können Rollen oder Förderbänder oder Gleitschienen. Derartige Mittel zur Beförderung der Paletten durch die in Reihe angeordneten Sterilisationscontainer ist besonders bevorzugt, wenn die in Reihe angeordneten Sterilisationscontainer in eine Produktionsstraße eingebunden sind. Dies ist wiederum insbesondere bevorzugt, wenn die in Reihe angeordneten Sterilisationscontainer in einen kontinuierlichen Prozess eingebunden werden sollen, d.h. die kontinuierlich fertiggestellten Paletten mit den zu sterilisierenden Produkten in einer Anzahl vor den in Reihe angeordneten Sterilisationscontainern gesammelt werden, wie sie danach in die Sterilisationskammer bzw. die Sterilisationskammern passen, dann mit Hilfe der Mittel zur Beförderung in die Sterilisationskammer bzw. in die Sterilisationskammern befördert werden und während der Dauer der Sterilisation wieder eine entsprechende Anzahl an Paletten vor dem ersten Sterilisationscontainer gesammelt wird, welche dann bei Entleerung der Sterilisationskammer bzw. der Sterilisationskammern durch den hintersten Sterilisationscontainer zeitgleich durch den vordersten Sterilisationscontainer in die Sterilisationskammer bzw. die Sterilisationskammern befördert werden. Damit ergibt sich ein kontinuierlicher Sterilisationsprozess. Durch Größe und Anzahl der in Reihe angeordneten Sterilisationscontainer, welche alle durch denselben Steuerungscontainer kontrolliert und betrieben werden können, ist diese Anlage flexibel einem geänderten Sterilisationsvolumen anpassbar. Es wird in obigen Ausführungen von einer einzigen Sterilisationskammer gesprochen, wenn die Sterilisationskammern der einzelnen in Reihe angeordneter Sterilisationscontainer zu einer einzigen Sterilisationskammer vereint wurden und es wird von mehreren Sterilisationskammern gesprochen, wenn die einzelnen Sterilisationskammern der einzelnen in Reihe angeordneter Sterilisationscontainer zwar zeitgleich aber als einzelne Sterilisationskammern betrieben werden. Beide Varianten sind möglich, ohne dass eine Variante bevorzugt ist. Alle Sterilisationskammern zu einer einzigen zu vereinen hat den Vorteil eines einfacheren Aufbaus und einer einfacherer Kontrolle der gesamten Sterilisationsanlage, wohingegen der parallele Betrieb der einzelnen Sterilisationskammern den Vorteil hat, Energie und Chemikalien zu sparen, wenn ausnahmsweise nicht alle Sterilisationskammern befüllt werden können und von beispielsweise 5 in Reihe angeordneter Sterilisationscontainer mit insgesamt 5 Sterilisationskammern nur zwei Sterilisationskammern benötigt werden.

Eine weitere bevorzugte Ausführungsform ist die parallele Anordnung von mehreren aber zumindest von 2 Sterilisationscontainern. Diese Anordnung ermöglicht die zeitgleiche Sterilisation unterschiedlicher Medizinprodukte sowie unterschiedlicher Mengen an Medizinprodukten bzw. an Paletten. Die parallele Anordnung der Sterilisationscontainern hat den Vorteil, dass die Sterilisationscontainer unabhängig voneinander und mit unterschiedlicher Befüllung betrieben werden können. Mit einer solchen Anlage lassen sich zeitgleich oder zeitversetzt verschiedenartige Medizinprodukte sterilisieren, wobei auch diese begehbare Sterilisationsanlage in eine Produktionsstraße eingebunden und die einzelnen Sterilisationskammern auch kontinuierlich betrieben werden können.

Die Sterilisation unterschiedlicher Medizinprodukte lässt sich aber auch mit in Reihe angeordneter Sterilisationscontainer mit separaten Sterilisationskammern, d.h. nicht zu einer Sterilisationskammer vereinter Sterilisationskammern der einzelnen Sterilisationscontainer erreichen, wenn die einzelnen Sterilisationskammern mit unterschiedlichen Produkten beladen und separat gesteuert werden.

Ein weiterer allgemeiner Aspekt der vorliegenden Erfindung betrifft die Zahl der **Zugänge** an dem Metallcontainer bzw. den Metallcontainern für Bedienpersonal bzw. für zu sterilisierende Produkte. Der Begriff "Zugang", wie hierin verwendet, steht für eine in der Regel verschließbare Verbindung zwischen dem Containerinnenraum und der äußeren Umgebung, über die das Bedienpersonal der Sterilisationsanlage in den Container gelangen kann. Bevorzugt wird, wenn diese Zugänge gasdicht verschließbar sind.

Wie bereits erwähnt, kann die erfindungsgemäße Ethylenoxid-Sterilisationsanlage in Container-Form in mindestens zwei Kompartimente unterteilt werden, die eigentliche Sterilisationskammer als ein Kompartiment und ein weiteres Kompartiment, welches auch als "Technikraum" bezeichnet werden kann, indem die weiteren technischen Komponenten der Anlage (wie z.B. Vakuumpumpe, Ethylenoxidverdampfer, Ethylenoxidtank, Steuerung, Befeuchtungsanlage, Lüftungsanlage) untergebracht sind. Diese Kompartimente können, wie bereits beschrieben, auf einen oder mehrere Container (vorzugsweise Metallcontainer) verteilt sein. Beide Kompartimente müssen natürlich von außen zugänglich sein, so dass die Sterilisationskammer beladen werden kann und im Technikraum leere Gasflaschen ausgetauscht oder Wartungsarbeiten durchgeführt werden können. Folglich weist die erfindungsgemäße Ethylenoxid-Sterilisationsanlage zumindest zwei Zugänge auf: mindestens einen zu der Sterilisationskammer und mindestens einen zu dem Technikraum. In der Ein-Container-Variante befinden sich diese mindestens zwei Zugänge an einem Container, in den Zwei- oder Mehr-Container-Varianten können diese Zugänge auf unterschiedliche Container verteilt sein. Zudem ist es möglich, dass diese Zugänge noch durch die Steuerung der Ethylenoxid-Sterilisationsanlage überwacht werden, d.h. die Stellung des Zuganges bzw. der Tür (also offen oder geschlossen) Tür wird erfasst, bzw. es wird von der Steuerung abgefragt, ob die Türen zum Container der Sterilisationskammer geschlossen sind, um eine effiziente Luftströmung innerhalb des Containers zu garantieren und eine Gefährdung der Mitarbeiter zu minimieren, d.h. ein Start der Anlage ist nur möglich, wenn keine Personen im Gefährdungsbereich sind. Ist innerhalb des Technikraums noch ein weiteres Kompartiment abgetrennt, das die Steuerung der Sterilisationsanlage beinhaltet, so ist dieses Kompartiment (auch als Kontrollraum oder Steuerungsraum bezeichnet) vorzugsweise durch einen separaten und damit dritten Zugang erreichbar. Dies hat zum Beispiel den Vorteil, dass im Fall einer Störung z.B. am Ethylenoxid-Verdampfer, das Bedienpersonal über den separat zugänglichen Steuerungsraum das System abschalten oder ein Notprogramm ausführen kann. Gemäß der Ein-Container-Variante ist es besonders bevorzugt, wenn sich der Zugang zu der Sterilisationskammer auf einer der Querseiten des Containers befindet, während sich der/die Zugänge zum Technik- bzw. Kontrollraum auf einer der Längsseiten des Containers befinden. In den Zwei-Container-Varianten sind zudem die Zugänge derart angeordnet, dass sie auch nach der funktionalen Verbindung beider Container noch zugänglich sind, d.h. vorzugsweise nicht auf den Containerseiten, die sich nach der Verbindung direkt gegenüber liegen.

Wie bereits erwähnt, muss natürlich die Sterilisationskammer zwingend von außen zugänglich sein, damit diese be- und entladen werden kann. Hier ist es bevorzugt, wenn sich der Zugang auf der Querseite des Metallcontainers befindet und sich zudem der Zugang im Wesentlichen über die gesamte Breite der Sterilisationskammer erstreckt. Dies erspart unnötige Rangiermanöver beim Be- und Entladen der Kammer und erhöht somit die Effizienz der Logistik. Der Begriff "im Wesentlichen über die gesamte Breite der Sterilisationskammer", wie hierin verwendet, bedeutet, dass die Breite der Zugangsöffnung (bzw. der Türöffnung) zu der Sterilisationskammer nicht mehr als 20 %, bevorzugt 18%, bevorzugt 15%, noch bevorzugter 12%, noch bevorzugter 10%, noch bevorzugter 8% und am meisten bevorzugt 5% kleiner ist als die Innenseite der Sterilisationskammer, in der sich der betreffende Zugang befindet.

Erfindungsgemäß wird besonders bevorzugt, wenn die Sterilisationskammer zwei Zugänge aufweist, welche sich zudem bevorzugt gegenüber liegen. Zwei Zugänge sind zum Beispiel für die Beladung der Sterilisationskammer mit den zu sterilisierenden Waren vorteilhaft, weil so die Sterilisationskammer von der einen Seite beladen und von der anderen Seite entladen werden kann. Dies ermöglicht es zum Beispiel bei Installation der erfindungsgemäßen Sterilisationsanlage in einen Gebäudekomplex oder in einen Lagerhallenkomplex, die Beladung und Entladung der Sterilisationskammer, ähnlich wie bei einer Sicherheitsschleuse, räumlich zu trennen. Dies hat nicht nur Vorteile für die Wahrung der Sterilität der Produkte, sondern kann überdies enorme logistische Vorteile haben.

Gemäß einer bevorzugten Ausführungsform der vorliegenden Erfindung besitzt der Metallcontainer zwei Zugänge zu der Sterilisationskammer, die sich an gegenüberliegenden Seiten des Containers befinden, besonders bevorzugt an den Querseiten des Containers. So ist es möglich, die Sterilisationskammer in ein automatisches Palettenfördersystem zu integrieren, so dass an dem ersten Zugang die Sterilisationskammer mit neuen zu sterilisierenden Paletten beladen wird und hierdurch gleichzeitig die bereits sterilisierten Paletten über den zweiten Zugang nach außen geschoben werden. Hierzu kann sich in der Sterilisationskammer eine an ein solches Fördersystem anschließbare Vorrichtung befinden, wie z.B. passive oder aktive Laufschienen oder Förderbänder. Es ist natürlich auch möglich (obwohl nicht bevorzugt), wenn sich die beiden Zugänge zu der Sterilisationskammer in senkrecht zueinander stehenden Seiten des Containers befinden.

Zusätzlich ist bevorzugt, wenn sich in der Sterilisationskammer der vorliegenden Erfindung ein oder mehrere Not-Aus-Schalter befinden (vorzugsweise in der Nähe des Zuganges bzw. der Zugänge), so dass Personen, die sich versehentlich in der Sterilisationskammer befinden, wenn die Zugänge geschlossen wurden, die Kammer abschalten und in Sicherheit gelangen können.

Zudem ist es möglich, dass die Lüftungsanlage derart konzipiert ist, dass bei geschlossenen Türen durch die Lüftungsanlage kein Unterdruck mehr erzeugt wird.

Dies würde versehentlich eingeschlossenen Personen den Austritt aus der Kammer erleichtern.
Diese Lüftungsöffnungen der Lüftungsanalage können bevorzugt in der Seitenwand oder der Decke der Sterilisationskammer integriert sein.

Die stationäre Ethylenoxid-Sterilisationsanlage dient vorzugsweise zur Sterilisation von Medizinprodukten und weiter bevorzugt zur Sterilisation von Medizinprodukten im Palettenverfahren. Eine manuelle Beladung mit einzelnen Medizinprodukten im Karton ist damit grundsätzlich auch möglich, wobei vorzugsweise Paletten verwendet werden.

Somit handelt es sich vorzugsweise bei der stationären Ethylenoxid-Sterilisationsanlage um eine Palettensterilisationsanlage, d.h. eine stationäre Ethylenoxid-Palettensterilisationsanlage.

Die vorliegende Erfindung betrifft daher eine begehbare stationäre Ethylenoxid-Palettensterilisationsanlage umfassend eine Vakuumpumpe, einen Ethylenoxidverdampfer, einen Ethylenoxidtank, eine Steuerung, eine Befeuchtungsanlage und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind.

Vorzugsweise sind in der begehbaren stationären Ethylenoxid-Palettensterilisationsanlage und in der Sterilisationskammer, d.h. in dem Metallcontainer, worin sich die Sterilisationskammer befindet und in der Sterilisationskammer selbst Mittel zur Beförderung der Paletten vorgesehen. Derartige Mittel können Rollen oder Förderbänder oder Gleitschienen sein, auf denen die Paletten bewegt werden. Vorzugsweise werden die Paletten mit diesen Mitteln durch den Metallcontainer entlang seiner Längsachse von einer Seite zur anderen und durch die Sterilisationskammer bewegt. Dementsprechend wird der Metallcontainer von einer Seite beladen und entsprechend von der gegenüberliegenden Seite entladen.

Gemäß einer weiteren Ausführungsform betrifft die vorliegende Erfindung somit ebenfalls eine begehbare stationäre Ethylenoxid-Sterilisationsanlage, wobei es sich bei der begehbaren stationären Ethylenoxid-Sterilisationsanlage um eine Palettensterilisationsanlage handelt und wobei in der begehbaren stationären Ethylenoxid-Sterilisationsanlage und in der Sterilisationskammer Mittel zur Beförderung der Paletten vorgesehen sind.

Diese Bewegung der Paletten durch den Metallcontainer und durch die Sterilisationskammer hindurch ermöglicht einen kontinuierlichen Durchlaufbetrieb der begehbaren stationären Ethylenoxid-Palettensterilisationsanlage. Dies ist vor allem dann vorteilhaft, wenn die begehbare stationäre Ethylenoxid-Sterilisationsanlage oder die begehbare stationäre Ethylenoxid-Palettensterilisationsanlage in eine Produktionsstraße eingebunden ist. Auf der Produktionsstraße werden die zu sterilisierenden Waren oder die Paletten mit den zu sterilisierenden Waren bewegt und passieren auf ihrem Weg dann auch die Sterilisationsanlage. Somit ist bevorzugt, wenn die begehbare stationäre Ethylenoxid-Sterilisationsanlage oder die begehbare stationäre Ethylenoxid-Palettensterilisationsanlage an eine Produktionsstraße anschließbar ist. Anschließbar bedeutet in diesem Zusammenhang, dass die Sterilisationsanlage in eine Produktionsstraße, insbesondere eine bestehende Produktionsstraße integrierbar ist. Dies könnte eine besondere Ausgestaltung des Metallcontainers dahingehend erfordern, dass Verbindungsmittel zu Fördermitteln der Produktionsstraße am Metallcontainer vorgesehen werden.

Gemäß einer weiteren Ausführungsform betrifft die vorliegende Erfindung somit ebenfalls eine begehbare stationäre Ethylenoxid-Sterilisationsanlage, wobei die begehbare stationäre Ethylenoxid-Sterilisationsanlage an eine Produktionsstraße anschließbar ist und/oder wobei die begehbare stationäre Ethylenoxid-Sterilisationsanlage kontinuierlich im Durchlaufbetrieb betreibbar ist.

Eine besonders bevorzugte Ausführungsform der vorliegenden Erfindung betrifft eine begehbare stationäre Ethylenoxid-Sterilisationsanlage und weiter bevorzugt eine begehbare stationäre Ethylenoxid-Palettensterilisationsanlage umfassend eine Vakuumpumpe, einen Ethylenoxidverdampfer, einen Ethylenoxidtank, eine Steuerung, eine Befeuchtungsanlage und eine Sterilisationskammer, dadurch gekennzeichnet, dass die Sterilisationskammer, die Vakuumpumpe, der Ethylenoxidverdampfer und der Ethylenoxidtank in mindestens einem Metallcontainer untergebracht sind und die Sterilisationskammer bei der Ein-Container-Variante ein Innenvolumen von mindestens 5% des Innenvolumens des Metallcontainers hat oder die Sterilisationskammer bei der Zwei-Container-Variante ein Innenvolumen von mindestens 35% des Innenvolumens des Metallcontainers hat, worin sich die Sterilisationskammer befindet. Bei dieser Ausführungsform sind ferner insbesondere die Mittel zur Beförderung der Paletten in der Sterilisationsanlage und der Sterilisationskammer vorteilhaft.

Wie bereits erwähnt, stellt der Begriff "stationär" fest, dass es sich nicht um eine tragbare EO-Sterilisationsanlage handelt, sondern um eine großtechnische Anlage, welche für die Sterilisation ganzer Paletten vorgesehen ist.

Es ist erfindungsgemäß bevorzugt, wenn die Sterilisationskammer der Ethylenoxid-Sterilisationsanlage mindestens 1 Europalette, bevorzugt mindestens 2 Europaletten, bevorzugter mindestens 4 Europaletten, weiter bevorzugt mindestens 6 Europaletten, noch weiter bevorzugt mindestens 8 Europaletten, bevorzugt mindestens 10 Europaletten, weiter bevorzugt mindestens 12 Europaletten, weiter bevorzugt mindestens 14 Europaletten, noch weiter bevorzugt mindestens 16 Europaletten, weiter bevorzugt mindestens 18 Europaletten, und am meisten bevorzugt mindestens 20 Europaletten gleichzeitig aufnehmen kann. Zudem ist eine Ethylenoxid-Sterilisationsanlage denkbar, deren Sterilisationskammer 36 Europlatten gleichzeitig aufnehmen kann (d.h. eine ganze LKW-Ladung).

Erfindungsgemäß bevorzugt ist eine Ethylenoxid-Sterilisationsanlage in einer Ein-Container-Variante, also eine Ethylenoxid-Sterilisationsanlage umfassend eine Vakuumpumpe, einen Ethylenoxidverdampfer, einen Ethylenoxidtank, eine Steuerung, eine Befeuchtungsanlage und eine Sterilisationskammer, dadurch gekennzeichnet, dass die Sterilisationskammer, die Vakuumpumpe, der Ethylenoxidverdampfer, der Ethylenoxidtank und die Befeuchtungsanlage in einem Metallcontainer untergebracht sind und die Steuerung in oder an demselben Metallcontainer angebracht ist.

Ebenfalls bevorzugt ist, eine Ein-Container-Variante, die zusätzlich eine Lüftungsanlage und/oder eine Abgasreinigungsanlage beinhaltet.

Eine noch weiter bevorzugte Ausführungsform der vorliegenden Erfindung betrifft daher eine Ethylenoxid-Sterilisationsanlage umfassend eine Vakuumpumpe, einen Ethylenoxidverdampfer, einen Ethylenoxidtank, eine Steuerung, eine Befeuchtungsanlage, eine Lüftungsanlage, eine Abgasreinigungsanlage und eine Sterilisationskammer, dadurch gekennzeichnet, dass die Sterilisationskammer, die Vakuumpumpe, der Ethylenoxidverdampfer, der Ethylenoxidtank, die Befeuchtungsanlage, die Lüftungsanlage sowie die Abgasreinigungsanlage in einem Metallcontainer untergebracht sind und die Steuerung in oder an demselben Metallcontainer angebracht ist.

Ein wesentlicher Vorteil der Ein-Container-Variante ist die weitgehende Autarkie der Ethylenoxid-Sterilisationsanlage. Alle wesentlichen Komponenten sind bereits im Transportzustand vormontiert, so dass die Ethylenoxid-Sterilisationsanlage nach Auslieferung an den Zielort lediglich unter minimalem Montageaufwand an die lokale Strom- und Wasserversorgung angeschlossen und mit Ethylenoxid-Tanks bestückt werden muss. Es müssen keine aufwändigen Verrohrungsarbeiten (besonders zwischen den explosionsgefährdeten Bauteilen) mehr stattfinden. Zudem ist die Anlage auf eine verbesserte Sicherheit ausgelegt und bedarf daher keiner umfangreichen Nachrüstungen am Zielort.

Wie bereits mehrfach erwähnt, kann die erfindungsgemäße Ethylenoxid-Sterilisationsanlage in zumindest zwei Kompartimente untergliedert werden, die eigentliche Sterilisationskammer und den Technikraum. In der Ein-Container-Variante ist es nun zum einen möglich, den Container (vorzugsweise ein normierter Metallcontainer) entlang einer zur Querseite des Containers parallel verlaufende Teilungslinie in Sterilisationskammer und Technikraum aufzuteilen. In diesem Zusammenhang wird auch von einer Teilung entlang der Querseite gesprochen. Zum anderen kann der Container entlang einer zur Längsseite des Containers parallel verlaufende Teilungslinie in Sterilisationskammer und Technikraum aufgeteilt werden. In diesem Zusammenhang wird auch von einer Teilung entlang der Längsseite gesprochen.

Erfolgt die Teilung des Containers (vorzugsweise des normierten Metallcontainers) in Sterilisationskammer und Technikraum entlang der Querseite des Containers, so ist bevorzugt, wenn ein Zugang zu der Sterilisationskammer auf der zur Sterilisationskammer gehörenden Querseite des Container liegt, wobei besonders bevorzugt ist, wenn sich zudem der Zugang zu der Sterilisationskammer im Wesentlichen über die gesamte Breite der Sterilisationskammer erstreckt. Ein weiterer Zugang zu der Sterilisationskammer kann sich auf der Längsseite des Containers befinden, wobei dann bevorzugt ist, wenn dieser Zugang möglich nah an der Teilungsline zum Technikraum liegt, also der Abstand zwischen beiden Zugängen zu der Sterilisationskammer möglichst groß ist. Es ist natürlich auch denkbar, dass sich jeweils ein Zugang zu der Sterilisationskammer auf jeder Längsseite des Containers (bzw. dem zu der Sterilisationskammer gehörenden Teil der Längsseite) befindet, wobei dann bevorzugt ist, wenn sich die Zugänge direkt gegenüber liegen. Der oder die Zugänge zum Technikraum können sowohl auf der zum Technikraum gehörenden Querseite des Containers als auch auf der Längsseite des Containers liegen. Erfolgt die Teilung des Containers in Sterilisationskammer und Technikraum entlang der Querseite des Containers, so ist zudem bevorzugt, wenn das Innenvolumen der Sterilisationskammer 5%, bevorzugt 7%, bevorzugt 9%, bevorzugt 10%, bevorzugt 12%, bevorzugt 15%, bevorzugt 18%, bevorzugt 20%, bevorzugt 25%, weiter bevorzugt 30%, noch weiter bevorzugt 35% und am meisten bevorzugt 40% des Innenvolumens des Containers einnimmt.

Erfolgt die Teilung des Containers (vorzugsweise des normierten Metallcontainers) in Sterilisationskammer und Technikraum entlang der Längsseite des Containers, so ist bevorzugt, wenn jeweils ein Zugang zu der Sterilisationskammer auf dem zur Sterilisationskammer gehörenden Abschnitt der beiden Querseiten des Container liegt. Hierbei ist besonders bevorzugt, wenn sich die Zugänge zu der Sterilisationskammer im Wesentlichen über die gesamte Breite der Sterilisationskammer erstrecken. Eine derartige Anordnung mit zwei gegenüberliegenden Zugängen zu der Sterilisationskammer bietet enorme logistische Vorteile, wie bereits oben ausgeführt worden ist. Es ist natürlich auch möglich, dass es nur einen Zugang zu der Sterilisationskammer gibt, wobei dies jedoch weniger bevorzugt ist.

Der oder die Zugänge zum Technikraum können auf den zum Technikraum gehörenden Abschnitten der Querseiten des Containers liegen. Es ist jedoch stärker bevorzugt, wenn sich der oder die Zugänge zum Technikraum auf der zum Technikraum gehörenden Längsseite des Containers befinden. Erfolgt die Teilung des Containers in Sterilisationskammer und Technikraum entlang der Längsseite des Containers, so ist zudem bevorzugt, wenn das Innenvolumen der Sterilisationskammer 25%, bevorzugt 27%, weiter bevorzugt 30%, noch weiter bevorzugt 32%, weiter bevorzugt 35%, weiter bevorzugt 37% und am meisten bevorzugt 40% des Innenvolumens des Containers einnimmt. Ein wichtiger Aspekt hierbei ist natürlich, dass bei einer Teilung des Containers entlang der Längsseite des Containers die resultierende Breite der Sterilisationskammer ausreichend ist, um zumindest eine Palette aufzunehmen (in Längsrichtung oder in Querrichtung). Daher ist bevorzugt, wenn bei einer Teilung des Containers entlang der Längsseite des Containers die resultierende Breite der Sterilisationskammer mindestens 65 cm, bevorzugt mindestens 70 cm, weiter bevorzugt mindestens 75 cm, weiter bevorzugt mindestens 80 cm, noch weiter bevorzugt mindestens 85 cm, weiter bevorzugt mindestens 90 cm, bevorzugt mindestens 95 cm, bevorzugt mindestens 100 cm, noch weiter bevorzugt mindestens 105 cm, weiter bevorzugt mindestens 110 cm, bevorzugt mindestens 115 cm, noch weiter bevorzugt mindestens 120 cm, und am meisten bevorzugt mindestens 125 cm beträgt.

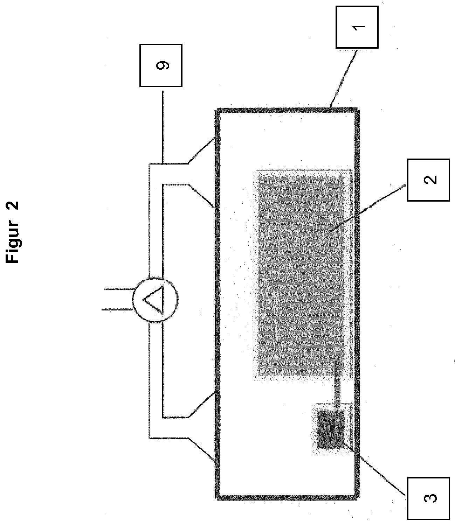

Figur 2 zeigt die Anbindung einer erfindungsgemäßen stationären Ethylenoxid-Sterilisationsanlage an eine Lüftungsanlage (9).

Zuluftleitungen als auch Abluftleitungen der Lüftungsanlage (9) können direkt mit dem Metallcontainer (1) verbunden werden und müssen nicht mehr für jeden einzelnen Raum vorgesehen werden, in dem sich ein Bauteil und insbesondere ein explosionsgefährdetes Bauteil der Sterilisationsanlage befindet. Durch die separate Containerraumluftabführung sind gebäudeseitige explosionsgeschützte Abluftanlagen der verschiedenen Sterilisationsbereiche daher nicht mehr notwendig.

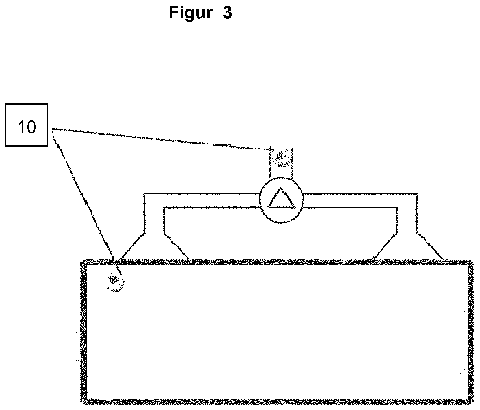

Figur 3 zeigt bevorzugte Stellen, wo Ethylenoxid-Sensoren (EO-Sensoren; 10) in der Anbindung der erfindungsgemäßen stationären Ethylenoxid-Sterilisationsanlage an eine Lüftungsanlage (9) vorgesehen werden können.

Grundsätzlich genügt ein Ethylenoxid-Sensor (10) in der Abluftleitung der Lüftungsanlage (9) oder innerhalb des Metallcontainers (1) (dargestellt in Figur 3 durch die beiden Punkte). Vom Metallcontainer (1) ist in Figur 3 nur der Umriss wiedergegeben, die Sterilisationsanlage im Inneren des Metallcontainers (1) ist in Figur 3 nicht gezeigt.

Die erfindungsgemäße stationäre Ethylenoxid-Sterilisationsanlage weist auch hinsichtlich der Überwachung der Ethylenoxid-Konzentration (also des EO-Monitorings) und hinsichtlich der Sicherheit in Bezug auf EO-Kontamination deutliche Vorteile auf. Bei den herkömmlichen Sterilisationsanlagen musste ein EO-Monitoring in allen Gebäuderäumen durchgeführt werden, wo Bauteile der Sterilisationsanlage untergebracht waren. Bei der erfindungsgemäßen stationären Ethylenoxid-Sterilisationsanlage beschränkt sich die Ethylenoxid-Überwachung auf den Containerinnenraum und/oder die Abluft. Bezogen auf die Überwachung der Ethylenoxid-Konzentration im Containerinnenraum ist es gemäß einer Ausführungsform der vorliegenden Erfindung bevorzugt, wenn sich im Containerinnerraum zumindest zwei Ethylenoxid-Sensoren (EO-Sensoren) befinden, wobei sich bevorzugt ein EO-Sensor in der Sterilisationskammer selbst befindet und sich ein weiterer EO-Sensor in dem Raum innerhalb des Containers aber außerhalb der Sterilisationskammer, also im so genannten Technikraum, befindet. In Ausführungsformen der vorliegenden Erfindung, in denen innerhalb des Technikraumes durch eine räumliche Abgrenzung ein weiteres Kompartiment abgegrenzt wird, in dem sich zum Beispiel die Steuerung der Ethylenoxid-Sterilisationslange, Befeuchtungsanlage und Lüftungsanlage befinden (siehe oben), so kann sich in diesem abgetrennten Bereich ein weiterer EO-Sensor befinden. Dies hat den Vorteil, dass die drei Kompartimente innerhalb des Containers, die aus unterschiedlichen Gründen von Personal betreten werden müssen (z.B. Be- und Entladung der Sterilisationskammer, Steuerung der Vorrichtung, Wartung der Geräte und Wechsel der Ethylenoxidtanks), unabhängig überwacht werden können, was gerade in Störfällen für die Sicherheit des Bedienungspersonal von enormer Bedeutung sein kann.

Durch die Containerbauweise verringert sich zudem das Raumvolumen und somit die Luftmenge für die notwendige Absaugung der Luft. Dies wiederum hat den Vorteil, dass die Lüftungsanlage kleiner dimensioniert werden kann.

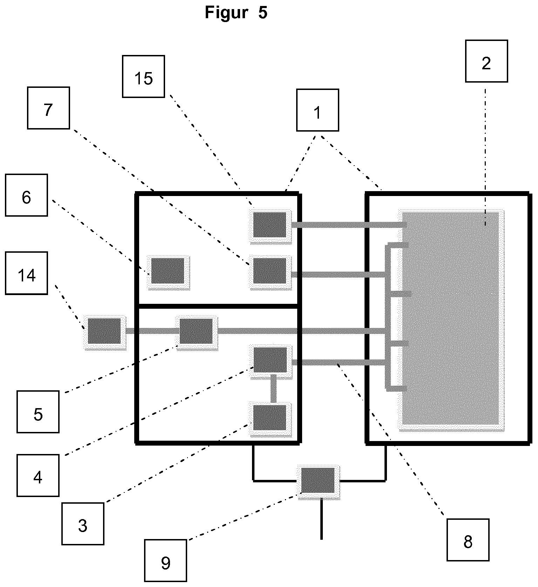

Figur 5 zeigt einen schematischen Aufbau einer erfindungsgemäßen stationären Ethylenoxid-Sterilisationsanlage in einer möglichen Zwei-Container-Variante umfassend eine Vakuumpumpe (5), einen Ethylenoxidverdampfer (4), einen Ethylenoxidtank (3), eine Entsorgungsanlage für das Ethylenoxid (EO-Entsorgung), also eine Abgasreinigungsanlage (14), welche sich außerhalb der Metallcontainer befindet, eine Steuerung oder Steuervorrichtung (6), eine Befeuchtungsanlage (7) in Form eines Dampfgenerators (7a), erforderliche Rohrleitungen (8) und eine Sterilisationskammer (2) sowie die außerhalb der Metallcontainer befindliche Lüftungsanlage (9). Die explosionsgefährdeten Bauteile umfassend die Sterilisationskammer (2), die Vakuumpumpe (5), den Ethylenoxidverdampfer (4) und den Ethylenoxidtank (3), wobei Vakuumpumpe (5), Ethylenoxidverdampfer (4) und Ethylenoxidtank (3) in einem ersten normierten Metallcontainer (1a) und die Sterilisationskammer (2) in einem damit verbundenen normierten zweiten Metallcontainer (1 b) untergebracht sind. Auch innerhalb des ersten Metallcontainers (1 a) aber in einem nicht zwingend explosionsgeschützten Bereich des ersten Metallcontainers (1 a) sind ferner Befeuchtungsanlage (7), Steuerung oder Steuervorrichtung (6) sowie ein Kammerheizung (z.B. Warmwassersystem) (15) vorgesehen.

Die Rohrleitungen (8) zwischen den beiden normierten Metallcontainern beschränken sich auf Leitungen zwischen der Kammerheizung (z.B. Warmwassersystem) (15) und der Sterilisationskammer (2), der Befeuchtungsanlage (7) und der Sterilisationskammer (2), der Vakuumpumpe (5) und der Sterilisationskammer (2) sowie dem Ethylenoxidverdampfer (4) und der Sterilisationskammer (2). Nahezu der vollständige Innenraum des normierten zweiten Metallcontainers wird für die Sterilisationskammer (2) zur Verfügung gestellt. Beide Metallcontainer und vorzugsweise Seecontainer werden bevorzugt in unmittelbarer Nähe zueinander aufgestellt und endmontiert. Die Lüftungsanlage (9) befindet sich zwangsläufig außerhalb der beiden Metallcontainer und ist mit den explosionsgeschützten Bereichen beider Metallcontainer verbunden.

### Referenzzeichenliste

- 1: Metallcontainer
- 1a: erster Metallcontainer
- 1b: zweiter Metallcontainer
- 2: Sterilisationskammer
- 3: Ethylenoxidtank oder Ethylenoxidflasche
- 4: Ethylenoxidverdampfer
- 5: Vakuumpumpe
- 6: Steuerung bzw. Steuervorrichtung
- 7: Befeuchtungsanlage
- 7a: Dampfgenerator
- 8: Rohrleitungen
- 9: Lüftungsanlage
- 10: Ethylenoxid-Sensor (EO-Sensor)
- 11: Waage
- 12: Anschluss für weitere Medien (z.B. Warmwasser)
- 13: räumliche Abtrennung
- 14: Abgasreinigungsanlage
- 15: Kammerheizung (z.B. Warmwassersystem)

### Figurenbeschreibung

**Figur 1**
   Figur 1 zeigt in Form eines stark vereinfachten Schemas eine erfindungsgemäße stationäre Ethylenoxid-Sterilisationsanlage umfassend eine Vakuumpumpe (5), einen Ethylenoxidverdampfer (4), einen Ethylenoxidtank (3), eine Steuerung oder Steuervorrichtung (6), einen Dampfgenerator (7a), eine Sterilisationskammer (2) und erforderliche Rohrleitungen (8). Es ist zu erkennen, dass die explosionsgefährdeten Bauteile, nämlich Vakuumpumpe (5), Ethylenoxidverdampfer (4), Ethylenoxidtank (3) und Sterilisationskammer (2) der stationären Ethylenoxid-Sterilisationsanlage in einem Metallcontainer (1) untergebracht sind und nur wenige Rohrleitungen durch die Containerwand führen. Die Steuerung oder Steuervorrichtung (6) ist außen am Metallcontainer (1) angebracht.
**Figur 2**
   Figur 2 zeigt in Form eines stark vereinfachten Schemas die Anbindung einer auf dem Metallcontainer (1) befindlichen erfindungsgemäßen stationären Ethylenoxid-Sterilisationsanlage an eine Lüftungsanlage (9). Ferner sind Sterilisationskammer (2) und Ethylenoxidtank (3) gezeigt.
**Figur 3**
   Figur 3 zeigt in Form eines stark vereinfachten Schemas bevorzugte Stellen, wo EO-Sensoren (10) in der Anbindung der erfindungsgemäßen stationären Ethylenoxid-Sterilisationsanlage an eine Lüftungsanlage (9) vorgesehen werden können.
**Figur 4**
   Figur 4 zeigt in Form eines stark vereinfachten Schemas eine erfindungsgemäße stationäre Ethylenoxid-Sterilisationsanlage umfassend eine Vakuumpumpe (5), einen Ethylenoxidverdampfer, (4) einen Ethylenoxidtank (3), eine Steuerung oder Steuervorrichtung (6), einen Dampfgenerator (7a), eine Sterilisationskammer (2) und erforderliche Rohrleitungen (8). Es ist zu erkennen, dass die explosionsgefährdeten Bauteile, nämlich Vakuumpumpe (5), Ethylenoxidverdampfer (4), Ethylenoxidtank (3) und Sterilisationskammer (2) der stationären Ethylenoxid-Sterilisationsanlage in einem Metallcontainer (1) untergebracht sind. Zudem sind in einem räumlich getrennten Bereich innerhalb des Metallcontainers (1) die Steuerung oder Steuervorrichtung (6) als auch der Dampfgenerator (7a) vorgesehen. Nur sehr wenige Rohrleitungen führen durch die Containerwand.
**Figur 5**
   Figur 5 zeigt in Form eines stark vereinfachten Schemas eine erfindungsgemäße stationäre Ethylenoxid-Sterilisationsanlage, wobei in einem normierten ersten Metallcontainer (1a) in einem abgetrennten explosionsgeschützten Raum dieses ersten Metallcontainers (1 a) Vakuumpumpe (5), Ethylenoxidverdampfer (4) und Ethylenoxidtank (3) vorgesehen sind und innerhalb dieses ersten Metallcontainers (1 a) in einem mittels einer räumlichen Abtrennung (13) davon abgetrennten Bereich Befeuchtungsanlage (7), Steuerung (6) und Kammerheizung (z.B. Warmwassersystem) (15) untergebracht sind. In räumlicher Nähe zu diesem ersten Metallcontainer (1 a) befindet sich ein normierter zweiter Metallcontainer (1b), worin die Sterilisationskammer (2) untergebracht ist.
**Figur 6**

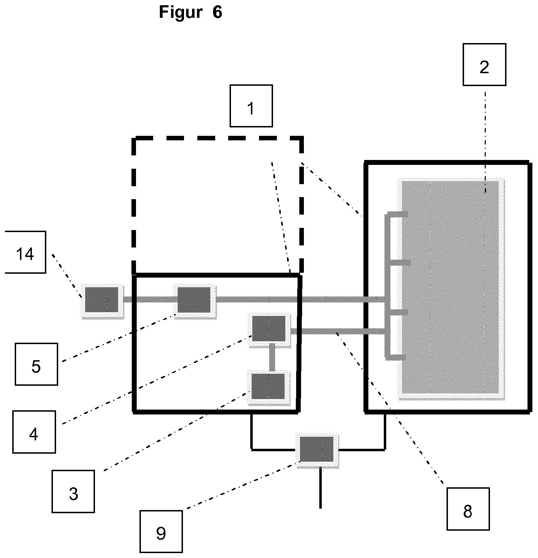

   Figur 6 zeigt in Form eines stark vereinfachten Schemas den Aufbau der stationären Ethylenoxid-Sterilisationsanlage gemäß Figur 5, wobei der nicht explosionsgeschützte Bereich des ersten Metallcontainers (1 a) durch gestrichelte Linien markiert ist.

## Patentansprüche

1. Begehbare stationäre Ethylenoxid-Sterilisationsanlage umfassend eine Vakuumpumpe (5), einen Ethylenoxidverdampfer (4), einen Ethylenoxidtank (3), eine Steuerung (6), eine Befeuchtungsanlage (7) und eine Sterilisationskammer (2), **dadurch gekennzeichnet, dass** die Sterilisationskammer (2), die Vakuumpumpe (5), der Ethylenoxidverdampfer (4) und der Ethylenoxidtank (3) in mindestens einem Metallcontainer (1) untergebracht sind.

2. Begehbare stationäre Ethylenoxid-Sterilisationsanlage gemäß Anspruch 1, wobei die Steuerung (6) außerhalb des mindestens einen Metallcontainers (1) oder von außen an dem mindestens einen Metallcontainer (1) angebracht ist.

3. Begehbare stationäre Ethylenoxid-Sterilisationsanlage gemäß Anspruch 1, wobei die Steuerung (6) in einem räumlich abgetrennten Bereich innerhalb des mindestens einen Metallcontainers (1) angebracht ist.

4. Begehbare stationäre Ethylenoxid-Sterilisationsanlage gemäß Anspruch 1, 2 oder 3, wobei es sich bei dem mindestens einen Metallcontainer (1) um einen Seecontainer handelt.

5. Begehbare stationäre Ethylenoxid-Sterilisationsanlage gemäß einem der Ansprüche 1 - 4, wobei die Vakuumpumpe (5), der Ethylenoxidverdampfer (3) und der Ethylenoxidtank (3) in einem ersten Metallcontainer (1 a) und die Sterilisationskammer (2) in einem zweiten Metallcontainer (1 b) untergebracht sind.

6. Begehbare stationäre Ethylenoxid-Sterilisationsanlage gemäß einem der Ansprüche 1 - 5, des Weiteren umfassend Rohrleitungen (8) zwischen Sterilisationskammer (2) und Vakuumpumpe (5), zwischen Sterilisationskammer (2) und Ethylenoxidverdampfer (4), zwischen Ethylenoxidverdampfer (4) und Ethylenoxidtank (3) sowie zwischen Sterilisationskammer (2) und Befeuchtungsanlage (7).

7. Begehbare stationäre Ethylenoxid-Sterilisationsanlage gemäß einem der Ansprüche 1 - 4 und 6, wobei sich die explosionsgefährdeten Bauteile innerhalb eines Metallcontainers (1) befinden.

8. Begehbare stationäre Ethylenoxid-Sterilisationsanlage gemäß einem der Ansprüche 1 - 7 des Weiteren umfassend eine Abgasreinigungsanlage (14) und eine Kammerheizung (15), wobei sich Abgasreinigungsanlage (14) und Kammerheizung (15) innerhalb des mindestens einen Metallcontainers (1) befinden.

9. Begehbare stationäre Ethylenoxid-Sterilisationsanlage gemäß einem der Ansprüche 1 - 8, wobei es sich bei der begehbaren stationären Ethylenoxid-Sterilisationsanlage um eine Palettensterilisationsanlage handelt.

10. Begehbare stationäre Ethylenoxid-Sterilisationsanlage gemäß Anspruch 9, wobei in der begehbaren stationären Ethylenoxid-Sterilisationsanlage und in der Sterilisationskammer (2) Mittel zur Beförderung der Paletten vorgesehen sind.

11. Begehbare stationäre Ethylenoxid-Sterilisationsanlage gemäß einem der Ansprüche 1 - 10, wobei die Sterilisationskammer (2) bei der Ein-Container-Variante ein Innenvolumen von mindestens 5% des Innenvolumens des Metallcontainers (1) hat oder wobei die Sterilisationskammer (2) bei der Zwei-Container-Variante ein Innenvolumen von mindestens 35% des Innenvolumens des Metallcontainers (1) hat, worin sich die Sterilisationskammer (2) befindet.

12. Begehbare stationäre Ethylenoxid-Sterilisationsanlage gemäß einem der Ansprüche 1 - 11, wobei die begehbare stationäre Ethylenoxid-Sterilisationsanlage an eine Produktionsstraße anschließbar ist und/oder wobei die begehbare stationäre Ethylenoxid-Sterilisationsanlage kontinuierlich im Durchlaufbetrieb betreibbar ist.

13. Begehbare stationäre Ethylenoxid-Sterilisationsanlage gemäß einem der Ansprüche 1 - 12, wobei die begehbare stationäre Ethylenoxid-Sterilisationsanlage zusätzlich eine Lüftungsanlage (9) zur Belüftung des/der Container (1) sowie der enthaltenen Sterilisationskammer (2) umfasst.

14. Begehbare stationäre Ethylenoxid-Sterilisationsanlage gemäß einem der Ansprüche 1 - 13, wobei die begehbare stationäre Ethylenoxid-Sterilisationsanlage zusätzlich Ethylenoxid-Sensoren (10) zur Überwachung der Ethylenoxid-Konzentration in der begehbaren stationären Ethylenoxid-Sterilisationsanlage sowie ein System zur Kontrolle des Öffnungszustandes von Zugängen zur Sterilisationskammer (2) umfasst, und wobei die Ethylenoxid-Sensoren (10) und das System zur Kontrolle des Öffnungszustandes von Zugängen zur Sterilisationskammer (2) an die Steuerung (6) angeschlossen sind.

15. Begehbare stationäre Ethylenoxid-Sterilisationsanlage gemäß einem der Ansprüche 1 - 14, in Gestalt eines modularen Systems umfassend einen ersten Metallcontainer (1 a) enthaltend eine Vakuumpumpe (5), einen Ethylenoxidverdampfer (4), einen Ethylenoxidtank (3), eine Steuerung (6) und eine Befeuchtungsanlage (7) sowie mindestens einen weiteren Metallcontainer (1 b) enthaltend eine Sterilisationskammer (2), wobei der erste Metallcontainer (1 a) verbindbar mit in der Größe variablen weiteren Metallcontainern (1 b) enthaltend eine Sterilisationskammer (2) ist.

## Claims

1. Walk-in stationary ethylene oxide sterilization system comprising a vacuum pump (5), an ethylene oxide evaporator (4), an ethylene oxide reservoir (3), a control (6), a humidifier (7) and a sterilization chamber (2), **characterized in that** the sterilization chamber (2), the vacuum pump (5), the ethylene oxide evaporator (4) and the ethylene oxide reservoir (3) are housed in at least one metal container (1).

2. Walk-in stationary ethylene oxide sterilization system according to Claim 1, wherein the control (6) is mounted outside the at least one metal container (1) or on the outside of the at least one metal container (1).

3. Walk-in stationary ethylene oxide sterilization system according to Claim 1, wherein the control (6) is mounted in a spatially separated area inside the at least one metal container (1).

4. Walk-in stationary ethylene oxide sterilization system according to Claim 1, 2 or 3, wherein the at least one metal container (1) is a sea container.

5. Walk-in stationary ethylene oxide sterilization system according to one of Claims 1 - 4, wherein the vacuum pump (5), the ethylene oxide evaporator (4), and the ethylene oxide reservoir (3) are housed in a first metal container (1a) and the sterilization chamber (2) is housed in a second metal container (1 b).

6. Walk-in stationary ethylene oxide sterilization system according to one of Claims 1 - 5, further comprising pipelines (8) between the sterilization chamber (2) and the vacuum pump (5), between the sterilization chamber (2) and the ethylene oxide evaporator (4), between the ethylene oxide evaporator (4) and the ethylene oxide reservoir (3) and between the sterilization chamber (2) and the humidifier (7).

7. Walk-in stationary ethylene oxide sterilization system according to one of Claims 1 - 4 and 6, wherein the potentially explosive components are accommodated inside a metal container (1).

8. Walk-in stationary ethylene oxide sterilization system according to one of Claims 1 - 7 further comprising an exhaust gas purification unit (14) and a chamber heating (15), wherein the exhaust gas purification unit (14) and the chamber heating (15) are accommodated inside at least one metal container (1).

9. Walk-in stationary ethylene oxide sterilization system according to one of Claims 1 - 8, wherein the walk-in stationary ethylene oxide sterilization system is a pallet sterilization system.

10. Walk-in stationary ethylene oxide sterilization system according to Claim 9, wherein means for the conveyance of the pallets are provided in the walk-in stationary ethylene oxide sterilization system and in the sterilization chamber (2).

11. Walk-in ethylene oxide sterilization system according to one of Claims 1 - 10, wherein the sterilization chamber (2) in the case of the single-container variant has an interior volume of at least 5% of the interior volume of the metal container (1) or wherein the sterilization chamber (2) in the case of the two-container variant has an interior volume of at least 35% of the interior volume of the metal container (1) in which the sterilization chamber (2) is accommodated.

12. Walk-in stationary ethylene oxide sterilization system according to one of Claims 1 - 11, wherein the walk-in stationary ethylene oxide sterilization system is connectable to a production line and/or wherein the walk-in stationary ethylene oxide sterilization system is capable of being operated on a continuous throughput basis.

13. Walk-in stationary ethylene oxide sterilization system according to one of Claims 1 - 12, wherein the walk-in stationary ethylene oxide sterilization system additionally comprises a ventilation unit (9) to ventilate the container or containers and the sterilization chamber (2) inside.

14. Walk-in stationary ethylene oxide sterilization system according to one of Claims 1 - 13, wherein the walk-in stationary ethylene oxide sterilization system additionally comprises ethylene oxide sensors (10) to monitor the ethylene oxide concentration in the walk-in stationary ethylene oxide sterilization system and a system for monitoring the open/close status of the access points to the sterilization chamber (2), and wherein the ethylene oxide sensors (10) and the system for monitoring the open/close status of the access points to the sterilization chamber (2) are connected to the control (6).

15. Walk-in stationary ethylene oxide sterilization system according to one of Claims 1 - 14, in the form of a modular system comprising a first metal container (1a) holding a vacuum pump (5), an ethylene oxide evaporator (4), an ethylene oxide reservoir (3), a control (6) and a humidifier (7) and at least another metal container (1 b) holding a sterilization chamber (2), wherein the first metal container (1a) can be connected to other metal containers (1 b) holding a sterilization chamber (2), these other metal containers (1 b) being variable in size.

## Revendications

1. Installation fixe et praticable de stérilisation à l'oxyde d'éthylène, comprenant une pompe à vide (5), un évaporateur d'oxyde d'éthylène (4), une cuve d'oxyde d'éthylène (3), une commande (6), une installation d'humidification (7) et une chambre de stérilisation (2), **caractérisée en ce que** la chambre de stérilisation (2), la pompe à vide (5), l'évaporateur d'oxyde d'éthylène (4) et la cuve d'oxyde d'éthylène (3), sont disposés dans au moins un récipient métallique (1).

2. Installation fixe et praticable de stérilisation à l'oxyde d'éthylène selon la revendication 1, dans laquelle la commande (6) est disposée à l'extérieur du ou des récipients métalliques (1) ou, de l'extérieur, contre le ou les récipients métalliques (1).

3. Installation fixe et praticable de stérilisation à l'oxyde d'éthylène selon la revendication 1, dans laquelle la commande (6) est disposée dans une zone spatialement distincte à l'intérieur du ou des récipients métalliques (1).

4. Installation fixe et praticable de stérilisation à l'oxyde d'éthylène selon la revendication 1, 2 ou 3, dans laquelle, pour ce qui concerne le ou les récipients métalliques (1), il s'agit d'un conteneur maritime.

5. Installation fixe et praticable de stérilisation à l'oxyde d'éthylène selon l'une des revendications 1 à 4, dans laquelle la pompe à vide (5), l'évaporateur d'oxyde d'éthylène (3) et la cuve d'oxyde d'éthylène (3) sont disposés dans un premier récipient métallique (1 a), et la chambre de stérilisation (2) dans un deuxième récipient métallique (1 b).

6. Installation fixe et praticable de stérilisation à l'oxyde d'éthylène selon l'une des revendications 1 à 5, comprenant en outre des tuyauteries (8) entre la chambre de stérilisation (2) et la pompe à vide (5), entre la chambre de stérilisation (2) et l'évaporateur d'oxyde d'éthylène (4), entre l'évaporateur d'oxyde d'éthylène (4) et la cuve d'oxyde d'éthylène (3), et entre la chambre de stérilisation (2) et l'installation d'humidification (7).

7. Installation fixe et praticable de stérilisation à l'oxyde d'éthylène selon l'une des revendications 1 à 4 et 6, dans laquelle des composants antidéflagrants se trouvent à l'intérieur d'un récipient métallique (1).

8. Installation fixe et praticable de stérilisation à l'oxyde d'éthylène selon l'une des revendications 1 à 7, comprenant en outre une installation (14) de purification des effluents gazeux et un chauffage (15) pour la chambre, l'installation (14) de purification des effluents gazeux et le chauffage (15) pour la chambre se trouvant à l'intérieur du ou des récipients métalliques (1).

9. Installation fixe et praticable de stérilisation à l'oxyde d'éthylène selon l'une des revendications 1 à 8, pour laquelle, pour ce qui concerne l'installation fixe et praticable de stérilisation à l'oxyde d'éthylène, il s'agit d'une installation de stérilisation à palettes.

10. Installation fixe et praticable de stérilisation à l'oxyde d'éthylène selon la revendication 9, dans laquelle, dans l'installation fixe et praticable de stérilisation à l'oxyde d'éthylène et dans la chambre de stérilisation (2), il est prévu des moyens pour faire avancer les palettes.

11. Installation fixe et praticable de stérilisation à l'oxyde d'éthylène selon l'une des revendications 1 à 10, dans laquelle la chambre de stérilisation (2), dans une variante à un récipient, a un volume intérieur d'au moins 5 % du volume intérieur du récipient métallique (1), ou dans laquelle la chambre de stérilisation (2), dans la variante à deux récipients, a un volume intérieur d'au moins 35 % du volume intérieur du récipient métallique (1) dans lequel se trouve la chambre de stérilisation (2).

12. Installation fixe et praticable de stérilisation à l'oxyde d'éthylène selon l'une des revendications 1 à 11, dans laquelle l'installation fixe et praticable de stérilisation à l'oxyde d'éthylène peut être raccordée à une ligne de production, et/ou dans laquelle l'installation fixe et praticable de stérilisation à l'oxyde d'éthylène peut être exploitée en continu en marche continue.

13. Installation fixe et praticable de stérilisation à l'oxyde d'éthylène selon l'une des revendications 1 à 12, dans laquelle l'installation fixe et praticable de stérilisation à l'oxyde d'éthylène comprend en outre une installation d'aération (9), pour aérer le ou les récipients (1), ainsi que la chambre de stérilisation qui y est contenue (2).

14. Installation fixe et praticable de stérilisation à l'oxyde d'éthylène selon l'une des revendications 1 à 13, dans laquelle l'installation fixe et praticable de stérilisation à l'oxyde d'éthylène comprend en outre des capteurs d'oxyde d'éthylène (10), pour surveiller la concentration de l'oxyde d'éthylène dans l'installation fixe et praticable de stérilisation à l'oxyde d'éthylène, ainsi qu'un système pour contrôler l'état d'ouverture des accès à la chambre de stérilisation (2), et dans laquelle les capteurs d'oxyde d'éthylène (10) et le système pour contrôler l'état d'ouverture des accès à la chambre de stérilisation (2) sont raccordés à la commande (6).

15. Installation fixe et praticable de stérilisation à l'oxyde d'éthylène selon l'une des revendications 1 à 14, sous forme d'un système modulaire comprenant un premier récipient métallique (1a) contenant une pompe à vide (5), un évaporateur d'oxyde d'éthylène (4), une cuve d'oxyde d'éthylène (3), une commande (6) et une installation d'humidification (7), ainsi qu'au moins un récipient métallique supplémentaire (1b) contenant une chambre de stérilisation (2), le premier récipient métallique (1a) pouvant être raccordé à des récipients métalliques supplémentaires (1 b), de taille variable, contenant une chambre de stérilisation (2).
